# EUROPEAN PATENT APPLICATION

(11) **EP 4 703 383 A1**
(43) Date of publication of application: **04.03.2026**
(21) Application number: 24795790.5
(22) Date of filing: 03.04.2024
(51) Int. Cl.: C07K 16/28, A61K 39/395

(54) **PROLACTIN RECEPTOR ANTIBODY AND USE THEREOF**

(30) Priority: 24.04.2023 CN 202310470408
(71) Applicant: Shenzhen Genuine Biomedical Technology Co., Ltd, Shenzhen, Guangdong 518000 (CN)
(72) Inventor: GUO, Changyue, Shenzhen, Guangdong 518000 (CN); CHEN, Lei, Shenzhen, Guangdong 518000 (CN); HE, Xiaoqin, Shenzhen, Guangdong 518000 (CN); LEI, Dan, Shenzhen, Guangdong 518000 (CN); TAN, Xiaoyu, Shenzhen, Guangdong 518000 (CN); XU, Mengying, Shenzhen, Guangdong 518000 (CN); DU, Jinfa, Shenzhen, Guangdong 518000 (CN)
(74) Representative: Cabinet Beau de Loménie
(86) International application number: PCT/CN2024/085827
(87) International publication number: WO 2024/222409

(57) **Abstract**

The present invention relates to a prolactin receptor antibody and the use thereof, and specifically relates to a PRLR-targeting antibody or an antigen-binding fragment thereof, or a variant having at least 85% sequence identity with the antibody or its antigen-binding fragment and retaining its PRLR binding activity, wherein the antibody targets the extracellular domain of the prolactin receptor. The provided antibody binds to PRLR with high affinity.

## Description

### FIELD OF THE INVENTION

The present invention relates to the fields of biology and medicine; more specifically, the present invention relates to an antibody specifically binding to a prolactin receptor and a use thereof.

### BACKGROUND OF THE INVENTION

Prolactin receptor (PRLR) is a single-pass transmembrane protein, a type I cytokine receptor, which is a homologous protein to interleukins 2, 3, 4, 6, 7, erythropoietin, and granulocyte macrophage colony stimulating factor receptor. The natural ligand of PRLR, prolactin, is a 199-amino-acid polypeptide hormone primarily secreted by lactotrophs in the human pituitary gland. After prolactin binds to the homodimeric form of prolactin receptor, it induces conformational changes in the receptor dimer, thereby activating Jak2 bound to the intracellular segment of the receptor. Jak2 catalyzes Stat5 to form an active phosphorylated dimer, and the phosphorylated Stat5 dimer enters the cell nucleus to regulate downstream gene expression. In addition to the main Jak2-Stat5 signaling pathway, the activation of prolactin receptor can also activate Src, promote cell proliferation, and activate MARK. In addition, growth factor hormones and placental lactogen can also bind to and activate prolactin receptors. Therefore, PRLR activation can mediate a variety of biological functions and is a potential target for disease intervention in certain benign diseases or malignant tumors.

Prolactin receptors mediate normal physiological functions including promoting cell growth, differentiation, development and lactation. Human prolactin receptor cDNA was originally isolated from liver cancer and breast cancer libraries. The mature protein has a full length of 598 amino acids. Due to different mRNA cutting, prolactin receptors have different subtypes, namely L, I, S1a and S1b. Several subtypes have the same extracellular segment and different lengths of intracellular segments. Prolactin receptors are considered to be potential therapeutic targets for breast cancer and prostate cancer, but their role in the occurrence and development of breast cancer has been controversial. In contrast, PRL synthesized outside the pituitary by autocrine and/or paracrine is believed to be involved in prostate tumorigenesis. In mouse models, overexpression of PRL in the prostate can lead to prostatic hyperplasia, intraepithelial neoplasia and even prostatic adenoma. From an epidemiological point of view, the expression of PRL and phosphorylated Stat5 in the prostate tissue of patients is positively correlated with the malignant grade of the tumor and the degree of disease invasion.

Prolactin receptors are not only potential targets for malignant tumors, but also important targets in a variety of benign lesions. Clinical studies have shown that enhanced PRLR-mediated signaling is associated with endometriosis in women. An exploratory clinical study showed that treatment with the dopamine agonist quinagolide (prolactin synthesis regulator) for more than 4 months in hyperprolactinemic women diagnosed with endometriosis can significantly reduce the size of peritoneal endometriotic lesions in female patients. In addition, prolactin can interfere with the hair follicle cycle, and increased prolactin concentrations in the blood are associated with hair loss. The expression of prolactin receptors in hair follicles has been demonstrated, and prolactin can induce the hair follicle cycle to enter the catagen phase. In mouse models, dopamine receptor agonists (PRL inhibitors) can promote hair growth in mice. In summary, PRLR is a potential drug target, and antibodies or antigen-binding fragments targeting PRLR can be used for a variety of PRLR-related diseases, with broad market prospects.

### SUMMARY OF THE INVENTION

The present invention first provides an antibody or an antigen-binding fragment thereof targeting PRLR, or a variant having at least 85% sequence identity with the antibody or antigen-binding fragment thereof and retaining the PRLR binding activity, wherein the antibody targets the extracellular domain of the prolactin receptor. Preferably, the extracellular domain includes amino acids 1-210 of SEQ ID NO: 99 or 101.

In one or more embodiments, the antibody comprises: 3 HCDRs of the heavy chain variable region shown by any one of SEQ ID NOs: 1-23, and/or 3 LCDRs of the light chain variable region shown by any one of SEQ ID NOs: 24-41. Preferably, the antibody comprises: 3 HCDRs of the heavy chain variable region shown by any one of SEQ ID NOs: 3, 5, 16, 20-23, and/or 3 LCDRs of the light chain variable region shown by any one of SEQ ID NOs: 26, 28, 36, 40, 41.

In one or more embodiments, the HCDR1 of the antibody comprises any one selected from SEQ ID NOs: 42-52, or a sequence having at least 85% sequence identity thereto.

In one or more embodiments, the HCDR2 of the antibody comprises any one selected from SEQ ID NOs: 53-66, or a sequence having at least 85% sequence identity thereto.

In one or more embodiments, the HCDR3 of the antibody comprises any one selected from SEQ ID NOs: 67-75, or a sequence having at least 85% sequence identity thereto.

In one or more embodiments, the LCDR1 of the antibody comprises any one selected from SEQ ID NOs: 76-83, or a sequence having at least 85% sequence identity thereto.

In one or more embodiments, the LCDR2 of the antibody comprises any one selected from SEQ ID NOs: 84-90, or a sequence having at least 85% sequence identity thereto.

In one or more embodiments, the LCDR3 of the antibody comprises any one selected from SEQ ID NOs: 91-98, or a sequence having at least 85% sequence identity thereto.

In one or more embodiments, the antibody comprises HCDR1, HCDR2, HCDR3 of an antibody shown in any one row of Table 1, or a sequence having at least 85% sequence identity thereto. In one or more embodiments, the antibody comprises LCDR1, LCDR2, LCDR3 of an antibody shown in any one row of Table 1, or a sequence having at least 85% sequence identity thereto.

In one or more embodiments, the HCDR1, HCDR2 and HCDR3 of the antibody are selected from any of the following groups or sequences having at least 85% sequence identity thereto:
(1) SEQ ID NO: 43, SEQ ID NO: 55, and SEQ ID NO: 67,
(2) SEQ ID NO: 43, SEQ ID NO: 66, and SEQ ID NO: 67,
(3) SEQ ID NO: 44, SEQ ID NO: 56, and SEQ ID NO: 68,
(4) SEQ ID NO: 50, SEQ ID NO: 63, and SEQ ID NO: 73, and/or
the LCDR1, LCDR2 and LCDR3 of the antibody are selected from any of the following groups or sequences having at least 85% sequence identity thereto:
(1) SEQ ID NO: 77, SEQ ID NO: 84, and SEQ ID NO: 91,
(2) SEQ ID NO: 82, SEQ ID NO: 90, and SEQ ID NO: 97.

In one or more embodiments, the antibody comprises HCDR1, HCDR2, HCDR3, LCDR1, LCDR2 and LCDR3 of an antibody shown in any one row of Table 1.

In one or more embodiments, the light chain variable region of the antibody comprises a mouse or human light chain FR region. In one or more embodiments, the heavy chain variable region of the antibody comprises a mouse or human light chain FR region.

In one or more embodiments, FR1, FR2, FR3 and FR4 of the heavy chain variable region of the antibody are each independently selected from FR1, FR2, FR3 and FR4 of the heavy chain variable region shown by any one of SEQ ID NOs: 1-23, and/or, FR1, FR2, FR3 and FR4 of the light chain variable region of the antibody are each independently selected from FR1, FR2, FR3 and FR4 of the light chain variable region shown by any one of SEQ ID NOs: 24-41.

In one or more embodiments, the FR region of the heavy chain variable region of the antibody is selected from the FR region of the heavy chain variable region shown by any one of SEQ ID NOs: 3, 5, 16, 20-23.

In one or more embodiments, the FR region of the light chain variable region of the antibody is selected from the FR region of the light chain variable region shown by any one of SEQ ID NOs: 26, 28, 36, 40, and 41.

In one or more embodiments, the antibody comprises VH, VL, or VH and VL of an antibody shown in any one row of Table 1, or a sequence having at least 85% sequence identity thereto.

In one or more embodiments, the VH of the antibody comprises the sequence as shown by any one of SEQ ID NOs: 3, 5, 16, 20-23, or a sequence having at least 85% sequence identity thereto. Alternatively or in addition, the VL of the antibody comprises the sequence as shown by any one of SEQ ID NOs: 26, 28, 36, 40, 41, or a sequence having at least 85% sequence identity thereto.

In one or more embodiments, the antibody:
VH is shown by SEQ ID NO: 3, and VL is shown by SEQ ID NO: 26;
VH is shown by SEQ ID NO: 5, and VL is shown by SEQ ID NO: 28;
VH is shown by SEQ ID NO: 16, and VL is shown by SEQ ID NO: 36;
VH is shown by SEQ ID NO: 20 or 21, and VL is shown by SEQ ID NO: 40;
VH is shown by SEQ ID NO: 22, and VL is shown by SEQ ID NO: 40;
VH is shown by SEQ ID NO: 23, and VL is shown by SEQ ID NO: 41;

In one or more embodiments, the antibody further comprises a heavy chain constant region and/or a light chain constant region.

In one or more embodiments, the antibody is a multispecific antibody, preferably a bispecific antibody.

In one or more embodiments, each antibody is a monoclonal antibody.

In one or more embodiments, the antibody is a chimeric antibody or a fully human antibody.

The present invention also provides a fusion protein or antibody conjugate, which comprises the antibody or antigen-binding fragment thereof described herein.

The invention also provides a polynucleotide, comprising a sequence selected from:
(1) the coding sequence of the antibody or antigen-binding fragment thereof, fusion protein, or antibody conjugate described in any embodiment of the present invention;
(2) a complement sequence of (1).

The present invention also provides a nucleic acid construct, which expresses the antibody or antigen-binding fragment thereof, fusion protein, or antibody conjugate described in any embodiment herein, or comprises the polynucleotide described in any embodiment herein.

In one or more embodiments, the nucleic acid construct is a vector, such as an integration vector, a cloning vector, or an expression vector.

The present invention also provides a phage comprising the antibody or antigen-binding fragment thereof described in any embodiment of the present invention or a library comprising the phage.

In one or more embodiments, the antibody or antigen-binding fragment thereof is displayed on the surface of the bacteriophage.

The present invention also provides a host cell, which:
(1) expressing and/or secreting the antibody or antigen-binding fragment thereof described in any embodiment of the present invention;
(2) comprising a polynucleotide as described herein; and/or
(3) comprising the nucleic acid construct described herein.

In one or more embodiments, the host cell is selected from a prokaryotic cell or a eukaryotic cell.

In one or more embodiments, the host cell is a mammalian cell.

The present invention also provides a method for producing an antibody or an antigen-binding fragment thereof, comprising: culturing the host cell described herein under conditions suitable for producing the antibody or the antigen-binding fragment thereof, and optionally purifying the antibody or the antigen-binding fragment thereof from the culture.

The present invention also provides a pharmaceutical composition, comprising the antibody or antigen-binding fragment thereof, fusion protein, antibody conjugate, polynucleotide, nucleic acid construct, phage or host cell described herein, and a pharmaceutically acceptable excipient.

In one or more embodiments, the auxiliary material is a vehicle, a diluent, or an excipient.

In one or more embodiments, the pharmaceutical composition is used for treating cancer.

In one or more embodiments, the cancer is a PRLR-related cancer. Preferably, the cancer is selected from ovarian cancer, melanoma, prostate cancer, intestinal cancer, gastric cancer, esophageal cancer, breast cancer, lung cancer, kidney cancer, pancreatic cancer, uterine cancer, liver cancer, bladder cancer, cervical cancer, oral cancer, brain cancer, testicular cancer, skin cancer, thyroid cancer and hematological malignancies. Hematological malignancies are selected from myeloma, chronic leukemia and acute leukemia.

The present invention also provides the use of the antibody or antigen-binding fragment thereof, fusion protein, antibody conjugate, polynucleotide, nucleic acid construct or host cell described in any embodiment herein in the preparation of a medicament for preventing or treating a disease.

In one or more embodiments, the disease includes: cancer, endometriosis, alopecia, osteoporosis, obesity, benign lesions caused by PRLR activation.

In one or more embodiments, the cancer is a PRLR-related cancer. Preferably, the cancer is selected from ovarian cancer, melanoma, prostate cancer, intestinal cancer, gastric cancer, esophageal cancer, breast cancer, lung cancer, kidney cancer, pancreatic cancer, uterine cancer, liver cancer, bladder cancer, cervical cancer, oral cancer, brain cancer, testicular cancer, skin cancer, thyroid cancer and hematological malignancies. Hematological malignancies are selected from myeloma, chronic leukemia and acute leukemia.

The present invention also provides a method for inhibiting the growth of tumor cells in a subject, inhibiting benign lesions caused by PRL activating PRLR, or treating or preventing a disease, the method comprising administering to a patient in need thereof a therapeutically effective amount of the antibody or antigen-binding fragment thereof, fusion protein, antibody conjugate or pharmaceutical composition described in any embodiment of the present invention.

In one or more embodiments, the disease includes: cancer, endometriosis, alopecia, osteoporosis, obesity, benign lesions caused by PRLR activation.

In one or more embodiments, the cancer is a PRLR-related cancer. Preferably, the cancer is selected from ovarian cancer, melanoma, prostate cancer, intestinal cancer, gastric cancer, esophageal cancer, breast cancer, lung cancer, kidney cancer, pancreatic cancer, uterine cancer, liver cancer, bladder cancer, cervical cancer, oral cancer, brain cancer, testicular cancer, skin cancer, thyroid cancer and hematological malignancies. Hematological malignancies are selected from myeloma, chronic leukemia and acute leukemia.

The present invention also provides a kit for detecting PRLR, which is used to evaluate the effect of drug treatment or diagnose cancer, and the kit comprises the antibody or its antigen-binding fragment, fusion protein, antibody conjugate, polynucleotide, nucleic acid construct, phage or host cell described in any embodiment of the present invention.

In one or more embodiments, the kit further comprises a reagent for detecting the binding of PRLR to an antibody or its antigen-binding fragment, fusion protein, or antibody conjugate. For example, the bound reagent is detected by the enzyme-linked immunosorbent assay.

In one or more embodiments, the detection reagent for binding is a detectable marker, such as biotin, that can be linked to an antibody or its antigen-binding fragment, a fusion protein, or an antibody conjugate. The detectable marker is attached to the antibody or its antigen-binding fragment or is separately present in the kit.

The present invention also provides a non-diagnostic method for detecting the presence of PRLR in a sample, comprising: incubating the sample with the antibody or its antigen-binding fragment, fusion protein, or antibody conjugate described in any embodiment of the present invention, and detecting the binding of PRLR to the antibody or its antigen-binding fragment, fusion protein, or antibody conjugate, thereby determining the presence of PRLR in the sample. The detection is an enzyme-linked immunosorbent assay.

The present invention also provides the use of the antibody or its antigen-binding fragment, fusion protein, or antibody conjugate described in any embodiment herein in the preparation of a kit for detecting PRLR in a sample, evaluating the effect of drug treatment, or diagnosing cancer.

### DESCRIPTION OF THE DRAWINGS

Figure 1 is an immunofluorescence detection of PRLR endocytosis mediated by the PRLR humanized antibody described herein.
Figure 2 is a detection of PRLR positive cell line mediated by the PRLR humanized antibody described herein.

### DETAILED DESCRIPTION

The practice of the present invention will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989); Oligonucleotide Synthesis (MJ. Gait, ed., 1984); Animal Cell Culture (RJ. Freshney, ed., 1987); Methods in Enzymology (Academic Press, Inc.); Current Protocols in Molecular Biology (F.M. Ausubel et al., eds 1987, and periodic updates); PCR: The Polymerase Chain Reaction, (Mullis et al., ed., 1994); A Practical Guide to Molecular Cloning (Perbal Bernard V., 1988); Phage Display: A Laboratory Manual (Barbas et al., 2001).

Prolactin receptor PRLR is a single transmembrane protein, a type I cytokine receptor, and homologous to receptors for interleukin2, 3, 4, 6, 7, erythropoietin, and granulocyte-macrophage colony-stimulating factor. Prolactin receptor can bind to human prolactin, growth factor hormones, and placental lactogen, mediating a variety of biological functions, including cell proliferation, differentiation, and lactation. Abnormal activation of prolactin receptors is associated with a variety of human diseases such as tumors, endometriosis, alopecia, hyperprolactinemia, and osteoporosis.

The present invention uses the extracellular segment of PRLR protein as an epitope peptide to obtain anti-human PRLR hybridoma monoclonal antibodies, and then uses phage display technology to screen the antibody gene library and perform humanization transformation, thereby obtaining a series of PRLR specific antibodies. Then, high-affinity, high-specificity, and high-functionally active antibodies are identified through methods such as ELISA, affinity, receptor internalization, blocking tests, and target cell killing. The antibody or antigen-binding fragment thereof has good safety and targeting, and can specifically bind to the extracellular domain of PRLR protein.

### Antibody

Term "antibody" described herein includes monoclonal antibodies (including full-length antibodies with immunoglobulin Fc region), antibody compositions with polyepitopic specificity, multi-specific antibodies (e.g., bispecific antibodies), diabodies and single chain molecules, and antibody fragments, especially antigen binding fragments, (e.g., Fab, F(ab')2, and FV). Herein, "antibody" and "immunoglobulin" are used interchangeably.

The traditional "antibody" contains the basic 4-chain antibody unit and it is a heterotetrameric glycoprotein composed of two identical light chains (L) and two identical heavy chains (H). Each heavy chain has a variable domain (VH) at the N-terminus, followed by three constant domains (for each α and γ chain, CH1, CH2, and CH3) and four constant domains (for µ and ε isoforms, CH1, CH2, CH3, and CH4) and the hinge region (Hinge) between the CH1 domain and the CH2 domain. Each light chain has a variable domain (VL) at the N-terminus, followed by a constant domain (CL) at the other end. The paired VH and VL together form an antigen binding site. For the structures and properties of different classes of antibodies, see, for example, Basic and Clinical Immunology, 8th Edition, Daniel P. Sties, Abba I. Terr, and Tristram G. Parsolw, ed, Appleton & Lange, Norwalk, CT, 1994, page 71, and Chapter 6. The L chain from any vertebrate species can be assigned to one of two clearly distinct types, called kappa and lambda, based on the amino acid sequences of their constant domains. The γ and α classes are further divided into subclasses on the basis of relatively minor differences in the CH sequence and function, e.g., humans express the following subclasses: IgGI, IgG2A, IgG2B, IgG3, IgG4, IgA1 and IgA2. The antibody heavy chain may further comprise a heavy chain constant region, comprising a constant region of IgG1, 2, 3, 4 of human or mouse origin or a mutant sequence thereof. The antibody light chain may further comprise a light chain constant region, comprising a constant region of a κ or λ chain of human or mouse origin or a mutant sequence thereof.

The "variable region" or "variable domain" of an antibody refers to the amino-terminal domains of the heavy or light chain of the antibody. The variable domains of the heavy chain and light chain may be referred to as "VH" and "VL", respectively. These domains are usually the most variable parts of the antibodies (relative to other antibodies of the same type) and contain the antigen binding sites.

The term "variable" refers to the fact that certain segments of the variable domains differ extensively in sequence among antibodies. Variable domains mediate antigen binding and define the specificity of a specific antibody to its specific antigen. However, variability is not evenly distributed across all amino acids spanned by the variable domain. Instead, it is concentrated in three segments called hypervariable regions (HVRs) both in the light-chain and the heavy chain variable domains. The more highly conserved part of the variable domain is called the framework regions (FR). The variable domains of native heavy and light chains each comprise four FR regions (FR1, FR2, FR3, and FR4), largely adopting a beta-sheet configuration, connected by three HVRs, which form loops connecting, and in some cases forming part of, the beta-sheet structure. The HVR in each chain is hold together by the FR region in close proximity, and contributes to the formation of the antigen binding site of the antibody together with the HVR of the other chain. Generally, the structure of the light chain variable region is FR1-LCDR1-FR2-LCDR2-FR3-LCDR3-FR4, and the structure of the heavy chain variable region is FR1-HCDR1-FR2-HCDR2-FR3-HCDR3-FR4. The constant domain is not directly involved in the binding of antibody to antigen, but exhibits a variety of effector functions, such as the involvement of antibody in antibodydependent cell-mediated toxicity. There are a variety of variable region labeling schemes for antibodies, including: Chothia, Kabat, IMGT, and Contact. The invention exemplarily uses the IMGT labeling scheme.

An "Fc region" (fragment crystallizable region) or "Fc domain" or "Fc" refers to the C-terminal region of the heavy chain of an antibody that mediates the binding of the immunoglobulin to host tissues or factors, including binding to Fc receptors located on various cells of the immune system (e.g., effector cells) or to the first component (Clq) of the classical complement system. In IgG, IgA and IgD antibody isotypes, the Fc region is composed of two identical protein fragments from the CH2 domain and CH3 domain of two heavy chains of the antibody; the Fc regions of IgM and IgE contain three heavy chain constant domains (CH domains 2-4) in each polypeptide chain. Although the boundary of the Fc region of an immunoglobulin heavy chain may vary, the Fc region of human IgG heavy chain is usually defined as a sequence segment from the amino acid residue of heavy chain position C226 or P230 to the carboxyl terminus, which is numbered according to the EU index, as in Kabat. As used herein, the Fc region may be a native sequence Fc or a variant Fc.

The term "antigen-binding fragment" generally refers to a portion of an antibody molecule comprising the amino acids responsible for the specific binding between the antibody and the antigen. The portion of an antigen that is specifically recognized and bound by an antibody is called an "epitope" as described above. As noted above, an antigen-binding domain may typically comprise an antibody light chain variable region (VL) and an antibody heavy chain variable region (VH); however, it need not necessarily comprise both. Examples of antibody fragments include Fab, Fab', F(ab'), F(ab')2, Fd, Fv and disulfide-linked Fv fragments; diabodies; linear antibodies; single-chain antibody molecules, scFv-Fc fragment; multispecific antibodies formed from antibody fragments; and any fragment that should be able to increase the half-life by chemical modification or by incorporation into liposomes. Fd fragments, for example, have two VH regions and typically retain some antigen-binding function of the full antigen-binding domain. Examples of antigen-binding fragments of antibodies include (1) Fab fragments, a monovalent fragments having VL, VH, constant light chain (CL) and CH1 domains; (2) F(ab')2 fragments, two Bivalent fragment having two Fab fragments connected by sulfur bridge; (3) Fd fragment with two VH and CH1 domains; (4) Fv fragment with VL and VH domains of antibody single arm, (5) dAb fragment (Ward et al., "Binding Activities of a Repertoire of Single Immunoglobulin Variable Domains Secreted From Escherichia coli," Nature 341:544-546 (1989), which is hereby incorporated by reference in its entirety), has a VH domain; (6) isolated complementarity determining regions (CDRs), and (7) single chain Fv (scFv), for example derived from scFv-libraries. Although the two domains VL and VH of the Fv fragment are encoded by separate genes, they can be joined using recombinant methods by a synthetic linker that allows it to be produced as a single protein in which the VL and VH regions pair to form a monovalent molecule chain (termed single-chain Fv (scFv)) (see, e.g., Huston et al., "Protein Engineering of Antibody Binding Sites: Recovery of Specific Activity in an Anti-Digoxin Single-Chain Fv Analogue Produced in Escherichia coli," Proc.Natl.Acad.Sci.USA 85:5879-5883 (1988)). These antibody fragments are obtained using conventional techniques known to those skilled in the art, and the function of the fragments can be evaluated in the same manner as intact antibodies.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site. "Single-chain Fv " also abbreviated as "sFv " or "scFv " are antibody fragments that comprise the VH and VL antibody domains connected into a single polypeptide chain. Preferably, the sFv polypeptide further comprises a polypeptide linker between the VH and VL domains which enables the sFv to form the desired structure for antigen binding. For heavy chain antibodies or nanobodies, the scFv is the VHH.

The term "monoclonal antibody" as used herein refers to an antibody obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method, phage-display technologies, recombinant DNA methods, and technologies for producing human or humanlike antibodies in animals that have parts or all of the human immunoglobulin loci or genes encoding human immunoglobulin sequences, single-cell sequencing methods.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity.

The term "murine antibody" in the present invention refers to a monoclonal antibody derived from mice that binds to human PRLR and is prepared according to the knowledge and skills in the art. During preparation, the test subject is injected with a PRLR antigen, and then the antibody expressed with the desired sequence or functional properties is separated. In a preferred embodiment of the present invention, the murine PRLR antibody or its antigen-binding fragment may further comprise a light chain constant region of a murine κ, λ chain or a mutant sequence thereof, or further comprise a heavy chain constant region of a murine IgG1, IgG2, IgG3 or a mutant sequence thereof.

The term "chimeric antibody" refers to an antibody formed by fusing the variable region of a murine antibody with the constant region of a human antibody, which can reduce the immune response induced by the murine antibody. To establish a chimeric antibody, it is necessary to first establish a hybridoma that secretes murine-specific monoclonal antibodies, then clone the variable region genes from the murine hybridoma cells, and then clone the constant region genes of human antibodies as needed. The murine variable region genes and the human constant region genes are connected into a chimeric gene and inserted into a vector, and finally the chimeric antibody molecules are expressed in eukaryotic cells or prokaryotic cells. In a preferred embodiment of the present invention, the antibody light chain of the chimeric antibody further comprises a light chain constant region of a human κ, λ chain or a mutant sequence thereof. The antibody heavy chain of the chimeric antibody further comprises a heavy chain constant region of a human IgG1, IgG2, IgG3 or IgG4 or a mutant sequence thereof, preferably comprises a human IgG1 heavy chain constant region.

"Humanized" forms of non-human (e.g., murine) antibodies are chimeric antibodies that contain minimal sequence derived from non-human immunoglobulin. Therefore, "humanized antibodies" generally refer to non-human antibodies that have had the variable- domain framework regions swapped for sequences found in human antibodies. Usually in a humanized antibody, the entire antibody (except CDRs) is encoded by or identical to a human derived polynucleotide (except CDRs). The CDRs, some or all of which are encoded by nucleic acids originating in a non-human organism, are grafted into the beta-sheet framework of a human antibody variable region to create an antibody, the specificity of which is determined by the engrafted CDRs. The term "humanized antibody" is also referred to as a CDR grafted antibody. The production methods of such antibodies are well known in the art, such as using mice with genetically engineered immune systems. Human germline antibody variable region framework (FR) sequences can be obtained from the website of ImMunoGeneTics (IMGT) (http://imgt.cines.fr.). The common sequence of human antibodies can be obtained from the website (https://plueckthun.bioc.uzh.ch/antibody/Modelling/HuCAL/index.html) (J.Mol.Biol.296, 57-86 (2000). In order to avoid the decrease in antibody activity caused by the decrease in immunogenicity, the variable region of the human antibody can be reversely mutated (back mutation) to maintain activity. In the present invention, the antibody includes a humanized variant.

A "human antibody" is an antibody that possesses an amino-acid sequence corresponding to that of an antibody produced by a human and/or has been made using any of the techniques for making human antibodies as disclosed herein. This definition of human antibodies clearly excludes a humanized antibody that contain non-human antigen binding residues. Human antibodies can be generated using a variety of techniques known in the art, including phage display libraries.

The term "binding to PRLR" in the present invention refers to being able to interact with human PRLR.

The term "antigen-binding epitope" of the present invention refers to a discrete three-dimensional site on an antigen that is recognized by the antibody or antigen-binding fragment of the present invention.

In some embodiments, the present invention also provides nanobodies, heavy chain antibodies, antibodies or antigen binding fragment thereof that compete with the antigen-binding region of any antibody of the present invention for binding to the same epitope on PRLR, that is, nanobodies, heavy chain antibodies, antibodies or antigen binding fragment thereof that can cross-compete with the antigen-binding region of any antibody of the present invention for binding to PRLR.

In a specific embodiment of the present invention, the anti-PRLR antibody contains the CDR group shown in any one row of Table 1:

**Table 1**

| Antibody No. | VH SEQ ID NO: | HCDR1 SEQ ID NO: | HCDR2 SEQ ID NO: | HCDR3 SEQ ID NO: | VL SEQ ID NO: | LCDR1 SEQ ID NO: | LCDR2 SEQ ID NO: | LCDR3 SEQ ID NO: |
|---|---|---|---|---|---|---|---|---|
| 1 | 1 | 42 | 53 | 67 | 24 | 76 | 84 | 91 |
| 2 | 2 | 42 | 54 | 67 | 25 | 77 | 84 | 92 |
| 3 | 3 | 43 | 55 | 67 | 26 | 77 | 84 | 91 |
| 4 | 4 | 42 | 53 | 67 | 27 | 77 | 85 | 92 |
| 5 | 5 | 44 | 56 | 68 | 28 | 77 | 84 | 91 |
| 6 | 6 | 42 | 53 | 67 | 28 | 77 | 84 | 91 |
| 7 | 7 | 45 | 57 | 67 | 29 | 77 | 84 | 92 |
| 8 | 8 | 42 | 54 | 67 | 28 | 77 | 84 | 91 |
| 9 | 9 | 46 | 58 | 69 | 30 | 78 | 86 | 93 |
| 10 | 10 | 43 | 59 | 67 | 28 | 77 | 84 | 91 |
| 11 | 11 | 42 | 53 | 67 | 28 | 77 | 84 | 91 |
| 12 | 4 | 42 | 53 | 67 | 25 | 77 | 84 | 92 |
| 13 | 7 | 45 | 57 | 67 | 31 | 77 | 84 | 92 |
| 14 | 3 | 43 | 55 | 67 | 31 | 77 | 84 | 92 |
| 15 | 6 | 42 | 53 | 67 | 28 | 77 | 84 | 91 |
| 16 | 12 | 43 | 55 | 67 | 32 | 79 | 87 | 92 |
| 17 | 13 | 47 | 60 | 70 | 33 | 80 | 88 | 94 |
| 18 | 14 | 48 | 61 | 71 | 34 | 81 | 89 | 95 |
| 19 | 15 | 49 | 62 | 72 | 35 | 80 | 88 | 96 |
| 20 | 16 | 50 | 63 | 73 | 36 | 82 | 90 | 97 |
| 21 | 17 | 48 | 61 | 71 | 37 | 81 | 89 | 95 |
| 22 | 18 | 51 | 64 | 74 | 38 | 83 | 90 | 98 |
| 23 | 19 | 52 | 65 | 75 | 39 | 83 | 90 | 98 |
| 24 | 20 | 43 | 55 | 67 | 40 | 77 | 84 | 91 |
| 25 | 21 | 43 | 66 | 67 | 40 | 77 | 84 | 91 |
| 26 | 22 | 44 | 56 | 68 | 40 | 77 | 84 | 91 |
| 27 | 23 | 50 | 63 | 73 | 41 | 82 | 90 | 97 |

The FR1, FR2, FR3 and FR4 of the antibody herein can be independently selected from the FR1, FR2, FR3 and FR4 of the VH or VL shown in any one row of Table 1. Preferably, in one or more embodiments, FR1, FR2, FR3 and FR4 of the heavy chain variable region of the antibody are each independently selected from FR1, FR2, FR3 and FR4 of the heavy chain variable region shown in any one of SEQ ID NOs: 1-23; FR1, FR2, FR3 and FR4 of the light chain variable region of the antibody are each independently selected from FR1, FR2, FR3 and FR4 of the light chain variable region shown by any one of SEQ ID NOs: 24-41.

In one or more embodiments, the antibody comprises VH, VL, or VH and VL of an antibody shown in any one row of Table 1. Preferably, VH is shown in SEQ ID NO: 3, VL is shown in SEQ ID NO: 26; or VH is shown in SEQ ID NO: 5, VL is shown in SEQ ID NO: 28; or VH is shown in SEQ ID NO: 16, VL is shown in SEQ ID NO: 36; or VH is shown in SEQ ID NO: 20 or 21, VL is shown in SEQ ID NO: 40; or VH is shown in SEQ ID NO: 22, VL is shown in SEQ ID NO: 40; or VH is shown in SEQ ID NO: 23, VL is shown in SEQ ID NO: 41.

In one or more embodiments, the antibody further comprises a heavy chain constant region and/or a light chain constant region. Exemplarily, the heavy chain of the antibody comprises the heavy chain constant region of human IgG1, IgG2, IgG3 or IgG4, or a sequence having at least 85% sequence identity therewith; the light chain of the antibody comprises the light chain constant region of human κ chain, λ chain, or a sequence having at least 85% sequence identity therewith. Preferably, the heavy chain of the antibody comprises the heavy chain constant region of human IgG1, or a sequence having at least 85% sequence identity therewith, and the light chain comprises the light chain constant region of human κ chain, or a sequence having at least 85% sequence identity therewith.

Herein, the antibody can be a multispecific antibody comprising one, two or more chains of antibodies described in the present invention or antigen binding fragments. The multispecificity can be against PRLR and another antigen, or it can be against two different epitopes of PRLR.

The present invention also comprises the antibody derivatives and analogues. "Derivatives" and "analogues" refer to polypeptides that basically maintain the same biological function or activity of the antibody of the present invention. The derivatives or analogues of the present invention may be polypeptides formed from (i) a polypeptide with a substituent group in one or more amino acid residues, or (ii) a polypeptide formed from fusion of a mature polypeptide with another compound, such as a compound that extends the half-life of the polypeptide, such as polyethylene glycol, or (iii) a polypeptide formed by fusing an additional amino acid sequence to this polypeptide sequence (such as a leader sequence or a secretory sequence, or a sequence or prokaryotic sequence used for purifying this polypeptide, or a fusion protein formed with a 6His tag). According to the teaching herein, these derivatives and analogues belong to common sense known to those skilled in the art.

The "mutation" in the "mutant sequence" described in the present invention includes, but is not limited to, "back mutation", "conservative modification" or "conservative substitution or replacement". The "conservative modification" or "conservative substitution or replacement" described in the present invention refers to the replacement of an amino acid in a protein with other amino acids having similar characteristics (e.g., charge, side chain size, hydrophobicity/hydrophilicity, main chain conformation and rigidity, etc.), so that changes can be made frequently without changing the biological activity of the protein. It is known to those skilled in the art that, in general, single amino acid replacement in a non-essential region of a polypeptide does not substantially change the biological activity (see, e.g., Watson et al. (1987) Molecular Biology of the Gene, The Benjamin/Cummings Pub. Co., p. 224, (4th edition)). In addition, replacement of amino acids with similar structures or functions is unlikely to destroy biological activity.

Without substantially affecting the activity of the antibody, those skilled in the art may change one or more (for example, 1, 2, 3, 4, 5, 6, 7, 8, 9 or 10 or more) amino acids to the of the antibody sequence of the invention to obtain the variant of the antibody or the functional fragment sequence thereof. These variants include (but are not limited to): deletion, insertion, and/or substitution of one or more (usually 1-50, preferably 1-30, more preferably 1-20, and most preferably 1-10) amino acids, and addition of one or more (usually less than 20, preferably less than 10, and more preferably less than 5) amino acids at the C-terminus and/or N-terminus. In this field, conservative substitution with amino acids with similar or similar properties usually does not change the function of the protein. For example, substituting with amino acids having similar properties may be performed in the FR and/or Fc regions. Amino acid residues available for conservative substitution are well known in the art. Such substituted amino acid residues may or may not be encoded by a genetic code. For another example, adding one or more amino acids to the C-terminus and/or N-terminus usually does not change the function of the protein. They are all considered to be included in the scope of the present invention.

The variant forms of the antibody described herein include: homologous sequence, conservative variant, allelic variant, natural mutant, induced mutant, protein encoded by DNA that can hybridize with the coding DNA of the antibody of the invention under high or low strictness conditions, and polypeptide or protein obtained by using the antiserum of the antibody of the invention. In some embodiments, the sequence of the variant of the present invention may have at least 95%, 96%, 97%, 98% or 99% identity with its source sequence. The sequence identity described in the invention can be measured using sequence analysis software. For example, the computer program BLAST with default parameters, especially BLASTP or TBLASTN. The invention also comprises those molecules with variable regions of antibody heavy chain with CDRs, if their CDRs have more than 90% homology (preferably more than 95%, more preferably more than 98%) with the CDRs identified here.

The antibody of present invention can be prepared by conventional methods in the art, such as hybridoma technology, phage display technology. Alternatively, the antibodies of the present invention can be expressed in other cell lines. Suitable mammalian host cells can be transformed with sequences encoding antibody of the invention, and then the host cells can be cultured and the antibody purified. Transformation can be carried out using any known method, including, for example, packaging polynucleotides in viruses (or viral vectors) and transducing host cells with the viruses (or vectors). The transformation procedure used depends on the host to be transformed. Methods for introducing heterologous polynucleotides into mammalian cells are well known in the art, including dextran mediated transfection, calcium phosphate precipitation, Polybrene mediated transfection, protoplast fusion, electroporation, encapsulation of polynucleotides in liposomes and direct microinjection of DNA into the nucleus. Mammalian cell lines that can be used as hosts for expression are well known in the art, including but not limited to a variety of immortalized cell lines available from the American Typical Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., HepG2), etc.

The present invention also provides a fusion protein comprising a PRLR extracellular region and a murine antibody IgG2a Fc fragment. The fusion protein is a protein product obtained by co-expressing two genes obtained by DNA recombination. The recombinant PRLR extracellular region Fc fusion protein is a fusion protein co-expressed by DNA recombination with the PRLR extracellular region and the murine antibody IgG2a Fc fragment. The recombinant PRLR extracellular region Fc fusion protein is a fusion protein co-expressed by DNA recombination with the PRLR extracellular region and the murine antibody IgG2a Fc fragment.

The recombinant PRLR extracellular region Fc fusion protein is a fusion protein co-expressed by DNA recombination with the PRLR extracellular region and the murine antibody IgG2a Fc fragment. In some embodiments, other polypeptides are located at the N-terminal and/or C-terminal of the antibody. In some embodiments, other polypeptides include polypeptides that locate antibodies to different organelles, labels for purification or labels for immune response, transmembrane proteins or their transmembrane regions, chimeric antigen receptors or their components (extracellular domains, hinge regions, transmembrane regions, signal transduction domains, costimulatory domains, etc.).

The present invention also includes an antibody conjugate containing the anti-PRLR antibody described herein, which is a class of drugs that a biologically active cytotoxic drug is connected to an antibody by a chemical bond, and the antibody is used as a carrier to target and transport the cytotoxic drug to a target cell to play a role. Those skilled in the art can select a drug coupled to the anti-PRLR antibody described herein according to needs and actual conditions.

### Nucleic acid

The present invention also provides polynucleotides encoding the antibodies described herein. The polynucleotide of the invention can be in the form of DNAs or RNAs. DNAs comprise cDNAs, genomic DNAs, or synthetic DNAs. DNAs can be single stranded DNAs or double stranded DNAs. DNAs can be coding or noncoding strand DNAs. The present invention also comprises degenerate variants of the polynucleotide sequence encoding the fusion protein, i.e., nucleotide sequences encoding the same amino acid sequence but with different nucleotide sequences. RNA may be mRNA that expresses antibodies in vivo and/or in vitro.

Therefore, the present invention also relates to polynucleotides that hybridize with the above polynucleotide sequences and have at least 50%, preferably at least 70%, more preferably at least 80% identity between the two sequences. The invention particularly relates to polynucleotides that can hybridize with the polynucleotides of the invention under strict conditions. In the present invention, "strict conditions" refer to: (1) hybridization and elution at lower ionic strength and higher temperature, such as 0.2 × SSC, 0.1 %SDS, 60 °C; or (2) addition of denaturants during hybridization, such as 50% (v/v) formamide, 0.1% calf serum/0.1% Ficoll, 42 °C, etc; or (3) hybridization that occurs only when the identity between two sequences is at least more than 90%, or preferably, more than 95%. Moreover, the polypeptides encoded by hybridizable polynucleotides have the same biological functions and activities as mature polypeptides.

The nucleotide full-length sequence of the antibody of the invention or fragment thereof can usually be obtained by PCR amplification method, recombination method or artificial synthesis method. A feasible method is to synthesize relevant sequences by artificial synthesis, especially with short fragment length. Usually, fragments with very long sequence can be obtained by synthesizing several small fragments first and then connecting them. In addition, the coding sequence of the heavy chain and the expression tag (such as 6His) can also be fused together to form a fusion protein. The sequences of the various parts of the fusion protein can be obtained as above and then connected to obtain the full length of the fusion protein.

Once the relevant sequences are obtained, they can be obtained in large quantities by recombination. They are related sequences usually cloned into vectors, transferred into cells, and then isolated from the proliferated host cells by conventional methods. The biomolecules (nucleic acids, proteins, etc.) according to the present invention include biomolecules in isolated form. At present, the DNA sequence encoding the protein (or fragment thereof, or derivative thereof) of the present disclosure can be obtained completely through chemical synthesis. This DNA sequence can then be introduced into various existing DNA molecules (or such as vectors) and cells known in the art. In addition, mutations can also be introduced into the protein sequence of the present disclosure through chemical synthesis. Each part of the fusion protein can be cloned sequentially into a vector or can be integrated into a full-length fusion protein and then cloned.

The present invention also relates to nucleic acid constructs containing the polynucleotide sequences described herein, and one or more regulatory sequences operably linked to these sequences, such as regulatory sequences suitable for expressing DNA or RNA as antibodies in vivo or in vitro. The polynucleotide sequence described herein can be manipulated in a variety of ways to ensure expression of the antibodies. The nucleic acid construct can be manipulated according to the difference or requirements of the expression vector prior to insertion of the nucleic acid construct into the vector. Techniques that utilize recombinant DNA methods to alter polynucleotide sequences are known in the art.

Regulatory sequences can be suitable promoter sequences. The promoter sequence is usually operably linked to the coding sequence of the protein to be expressed. The promoter can be any nucleotide sequence that shows transcriptional activity in the host cell of choice, comprising mutated, truncated, and hybrid promoters, and can be derived from genes encoding extracellular or intracellular polypeptides homologous or heterologous to the host cell. An example of a suitable promoter is the immediate early cytomegalovirus (CMV) promoter sequence. The promoter sequence is a strong constitutive promoter sequence capable of driving high-level expression of any polynucleotide sequence operably linked thereto. Another example of a suitable promoter is elongation growth factor-1α (EF-1α). However, other constitutive promoter sequences can also be used, including but not limited to the early stage of simian virus 40 (SV40) promoter, mouse mammary tumor virus (MMTV), human immunodeficiency virus (HIV) long terminal repeat (LTR) promoter, MoMuLV promoter, avian leukemia virus promoter, Epstein-Barr virus immediate early promoter, Rous sarcoma virus promoters, as well as human gene promoters, such as, but not limited to, actin promoter, myosin promoter, heme promoter, and creatine kinase promoter. Furthermore, the use of inducible promoters may also be considered. The use of inducible promoters provides a molecular switch capable of turning on expression of a polynucleotide sequence operably linked to the inducible promoter when expression is required and turning off expression when expression is undesirable. Examples of inducible promoters include but are not limited to metallothionein promoters, glucocorticoid promoters, progesterone promoters, and tetracycline promoters.

The regulatory sequence can also be a suitable transcription termination sequence, a sequence recognized by a host cell to terminate transcription. A terminator sequence is operably linked to the 3' terminal of the nucleotide sequence encoding the polypeptide. Any terminator that is functional in the host cell of choice can be used in the present invention. The regulatory sequence can also be a suitable leader sequence, an untranslated region of the mRNA important for translation by the host cell. A leader sequence is operably linked to the 5' terminal of the nucleotide sequence encoding the polypeptide. Any terminator that is functional in the host cell of choice can be used in the present invention.

Other regulatory sequences suitable for expressing DNA or RNA in vivo or in vitro are common knowledge in the art.

In some embodiments, the nucleic acid construct described herein is a cloning vector, such as a cloning vector, an expression vector and an integration vector. Expression of the present polynucleotide sequences is generally achieved by operably ligating the polynucleotide sequence of the invention to an expression vector. A typical cloning vector comprises transcriptional and translational terminators, initiation sequences and a promoter useful for regulating the expression of the desired nucleic acid sequence. Integrating vectors contain components for integrating the target sequence into the genome of the cell. These vectors can be used to transform appropriate host cells to enable them to express proteins. Vectors usually contain sequences for plasmid maintenance and for cloning and expressing exogenous nucleotide sequences. The sequences (collectively referred to as "flanking sequence" in some embodiments) generally comprises one or more of the following nucleotide sequences: promoter, one or more enhancer sequences, replication origin, transcription termination sequence, complete intronic sequence comprising donor and receptor splice sites, sequence encoding leader sequence for polypeptide secretion, ribosome binding site, polyadenylation sequence, a multilinker region for inserting nucleic acids encoding antibodies to be expressed and an optional marker element. Examples of expression vectors are pcDNA3.1.

In addition, the type of vector is not limited, for example, plasmids, phagemids, phage derivatives, animal viruses, and cosmids, which may vary depending on the host cell to be introduced. Viral vector technology is well known in the art and described, for example, in Sambrook et al. (2001, Molecular Cloning: A Laboratory Manual, Cold Spring Harbor Laboratory, New York) and other handbooks of virology and molecular biology. Viruses that can be used as vectors include, but are not limited to, retroviruses, adenoviruses, adeno-associated viruses, herpesviruses, and lentiviruses.

To assess the expression of a polypeptide or portion thereof, the vector introduced into the cell can also comprise either or both of a selectable marker gene or a reporter gene to facilitate identification and selection of expressing cells accessed from a cluster of transfected or infected cells by the viral vector.

### Cell

The host cells suitable for introducing the nucleic acid constructs described herein can be can be prokaryotic cells, such as bacterial cells; or lower eukaryotic cells, such as yeast cells; or higher eukaryotic cells, such as mammalian cells. Representative examples are: Escherichia coli, Streptomyces; bacterial cells of Salmonella typhimurium; fungal cells such as yeast; insect cells of Drosophila S2 or Sf9; animal cells of CHO, COS7, 293 cells, etc. Examples of mammalian cells include immune cells, preferably immune effector cells. "Immune effector cells" are immune cells that can perform immune effector functions, including: T cells, NK cells, peripheral blood mononuclear cells (PBMC), neutrophils, eosinophils, hematopoietic stem cells. T cells suitable for use in the present invention can be various types of T cells from various sources.

Methods for introducing nucleic acids or vectors into mammalian cells are known in the art, and the vectors may be transferred into cells by physical, chemical or biological methods. When the host is a prokaryote such as Escherichia coli, competent cells that can absorb DNA can be harvested after the exponential growth period and treated with CaCl₂ method, the steps of which are well known in the art. When the host is eukaryote, the following DNA transfection methods can be selected: calcium phosphate coprecipitation method, conventional mechanical methods such as microinjection, electroporation, liposome packaging, etc. In some embodiments, transduced or transfected immune effector cells proliferate ex vivo after introducing nucleic acids or vectors.

The obtained transformants can be cultured by conventional methods to express the antibody encoded by the gene of the invention. According to the host cells used, the medium used in the culture can be selected from various conventional media. Culture is performed under conditions suitable for host cell growth. When the host cells grow to the appropriate cell density, the selected promoters are induced by appropriate methods (such as temperature conversion or chemical induction), and the cells are cultured for another period of time.

The polypeptide in the above method can be expressed inside the cell, on the cell membrane, or secreted outside the cell. If necessary, the recombinant protein can be separated and purified by various separation methods using its physical, chemical, and other characteristics. These methods are familiar to those skilled in the art. Examples of these methods include but are not limited to: conventional renaturation treatment, treatment with protein precipitant (salting-out method), centrifugation, permeation, ultra-treatment, ultra-centrifugation, molecular sieve chromatography (gel filtration), adsorption chromatography, ion exchange chromatography, high-performance liquid chromatography (HPLC) and other various liquid chromatography technologies and combinations of these methods. For example, Protein A or G Sepharose FF column is used to pass the column, nonspecific binding components are washed away, and then the bound antibodies are eluted with an acidic buffer, and the antibody fragments are detected by SDS-PAGE, and the collected antibodies can be filtered and concentrated by conventional methods. Soluble mixtures and polymers can also be removed by conventional methods, such as gel filtration chromatography and ion exchange. The obtained product needs to be immediately frozen, such as 80°C, or freeze-dried.

### Uses and Methods

By constructing a nanoantibody library, the inventors screened nanoantibody and variants thereof that could bind PRLR. The binding of these antibodies to antigen was verified by protein-level binding assays, affinity assays, competitive blocking experiments, and tissue cross-reactions.

All aspects of the antibodies, coding sequences, nucleic acid constructs, and cells described herein can be used to prepare medicaments for preventing or treating the various conditions and diseases described herein, which are diseases or conditions associated with PRLR expression, referring to diseases caused directly or indirectly by abnormal PRLR expression, generally referring to diseases caused by overexpression of PRLR, such as cancer, including but not limited to: acute lymphocytic leukemia (ALL), chronic lymphocytic leukemia (CLL) and B-cell lymphoma, such as relapsed or refractory acute lymphocytic leukemia (r/r ALL); relapsed or refractory diffuse large B-cell lymphoma (r/r DLBCL); relapsed or refractory follicular lymphoma (r/r FL), etc.

The present invention also provides a pharmaceutical composition, which contains any one or more of antibodies or antigen-binding fragments thereof, fusion proteins, nucleic acid molecules, nucleic acid constructs and cells, and pharmaceutically acceptable excipients.

The antibodies, nucleic acids or CAR-modified cells of the present invention can be used alone or as pharmaceutical compositions in combination with diluents and/or with other components such as relevant cytokines or cell groups. In this regard, pharmaceutical compositions can be prepared by admixing the active agent having the desired degree of purity, optionally with a pharmaceutically acceptable carrier, as a lyophilized formulation or as an aqueous solution. Pharmaceutically acceptable vehicles are non-toxic to recipients at the dosages and concentrations employed and can include at least one of buffering agents (e.g., neutral buffered saline, sulfate buffered saline), antioxidants, preservatives, isotonic agents, stabilizing agents, a chelating agent (such as EDTA or glutathione), an adjuvant (such as aluminum hydroxide) and a surfactant. Furthermore, in order for pharmaceutical compositions to be used in vivo administration, they must be sterile. Pharmaceutical compositions can be rendered sterile by filtration through sterile filtration membranes.

In some embodiments, the pharmaceutical composition may contain at least one additive of cytotoxic agents, chemotherapeutic agents, cytokines, immunosuppressants, growth inhibitors, and active agents as required for the particular indication being treated. The specific addition amount of additives can be adjusted according to actual needs.

The pharmaceutical composition of the present invention can be administered in an amount of "immunologically effective amount", "anti-tumor effective amount", "tumor-inhibitory effective amount" or "therapeutic amount". "Treatment" refers to the subject accepting the treatment scheme described herein to achieve at least one positive treatment effect (for example, a reduced number of cancer cells, a reduced tumor volume, a reduced rate of cancer cells infiltrating into peripheral organs, or a reduced rate of tumor metastasis or tumor growth). When an "immunologically effective amount", "anti-tumor effective amount", "tumor-inhibitory effective amount" or "therapeutic amount" is indicated, the precise amount of the composition of the present application to be administered can be determined by a physician, taking into account patients' (subjects') age, weight, tumor size, extent of infection or metastasis, and individual differences in the condition. Generally, pharmaceutical compositions comprising T cells described herein may be administered at a dose of 10⁴ to 10⁹ cells/kg body weight, preferably 10⁵ to 10⁶ cells/kg body weight. T cell compositions can also be administered multiple times at these dosages. Cells can be administered using infusion techniques well known in immunotherapy (see, e.g., Rosenberg et al., New Eng. J. of Med. 319:1676, 1988). The optimal dosage and treatment regimen for a particular patient can be readily determined by one skilled in the medical field by monitoring the patient for signs of disease and adjusting treatment accordingly.

Administration of compositions may be in any convenient manner, including by spraying, injection, swallowing, infusion, implantation or transplantation. The compositions as used herein can be administered to a patient subcutaneously, intradermally, intratumorally, intranodally, intraspinally, intramuscularly, by intravenous injection, or intraperitoneally. The T cell composition can be injected directly into tumors, lymph nodes, or sites of infection.

In some embodiments of the present invention, the compositions thereof in the present invention can be combined with other therapies known in the art. Such therapies include, but are not limited to, chemotherapy, radiotherapy, and immunosuppressants. For example, the compositions of the present invention may be combined with radiotherapy or chemotherapy agents known in the art to treat PRLR-mediated diseases.

Herein, "anti-tumor effect" refers to a biological effect, which can be expressed by a reduction in tumor volume, a reduction in the number of tumor cells, a reduction in the number of metastases, an increase in life expectancy, or an improvement in various physiological symptoms related to cancer.

"Patient," "subject," "individual" and the like are used interchangeably herein to refer to a living organism, such as a mammal, that can elicit an immune response. Examples include, but are not limited to, humans, dogs, cats, mice, rats, and transgenic species thereof.

The present invention is described in further detail with reference to the following experimental examples. These examples are provided for illustrative purposes only and are not intended to be limiting unless otherwise specified. Accordingly, the present invention should in no way be construed as limited to the following examples, but should be construed to include any and all changes that become apparent in light of the teachings provided herein. Unless otherwise specified, the methods and materials used in the examples are conventional materials and methods in the art.

### Diagnosis, detection, and kit

The antibodies of the present invention can be used for determinations due to their high affinity to PRLR, such as combined determinations to detect and/or quantify PRLR expressed in tissues or cells. The antibodies can be used in further studies investigating the role of PRLR in disease. The methods for detecting PRLR are generally as follows: obtaining cell and/or tissue samples; and detecting the level of PRLR in the sample.

The PRLR antibody of the invention can be used for diagnostic purposes to detect, diagnose or monitor diseases and/or conditions associated with PRLR. The present invention provides the detection of the presence of PRLR in a sample using a classical immunohistological method known to those skilled in the art. Detection of PRLR can be performed in vivo or in vitro. Examples of methods suitable for detecting the presence of PRLR include ELISA, FACS, RIA, and the like.

For diagnostic applications, the antibody is typically labeled with a detectable labeling group. Suitable labeling groups include (but are not limited to): radioisotopes or radionuclides (e.g., 3H, 14C, 15N, 35S, 90Y, 99Tc, 111In, 125I, 1311), fluorescent groups (e.g., FITC, rhodamine, lanthanide phosphors), enzymatic groups (e.g., horseradish peroxidase, β-galactosidase, luciferase, alkaline phosphatase), chemiluminescent groups, biotinyl groups, or predetermined polypeptide epitopes recognized by secondary reporters (e.g., leucine zipper pair sequences, binding sites for secondary antibodies, metal binding domains, epitope tags), MRI (magnetic resonance imaging) or CT (computed tomography) contrast agents. Various methods for labeling proteins are known in the art and can be used to carry out the present invention.

Another aspect of the present invention provides a method for detecting the presence of a test molecule that competes with an antibody of the present invention to bind PRLR. An example of one such assay would involve detecting the amount of free antibody in a solution containing an amount of PRLR in the presence or absence of the test molecule. An increase in the amount of free antibody (i.e., antibody that does not bind PRLR) will indicate that the test molecule can compete with the antibody for binding to PRLR. In one embodiment, the antibody is labeled with a labeling group. Alternatively, the test molecule is labeled, and the amount of free test molecule is monitored in the presence or absence of the antibody.

The present invention also provides a detection kit for detecting PRLR level. The kit comprises a PRLR antibody, a lysis medium for dissolving samples, and general reagents and buffers required for detection, such as various buffers, detection markers, detection substrates, etc. The detection kit can be an in vitro diagnostic device.

### Some specific implementation plans

1. An antibody or an antigen-binding fragment thereof targeting PRLR, or a variant having at least 85% sequence identity with the antibody or its antigen-binding fragment thereof and retaining the PRLR binding activity, wherein the antibody targets the extracellular domain of the prolactin receptor,
   preferably, the extracellular domain includes amino acids 1 to 210 of SEQ ID NO: 99 or 101,
   more preferably, the antibody comprises: three HCDRs of the heavy chain variable region shown by any one of SEQ ID NOs: 1 to 23, and/or three LCDRs of the light chain variable region shown by any one of SEQ ID NOs: 24 to 41.
2. The antibody or antigen-binding fragment thereof according to item 1, wherein
   the HCDR1 of the antibody comprises any one selected from SEQ ID NOs: 42 to 52, or a sequence having at least 85% sequence identity thereto, and/or
   the HCDR2 of the antibody comprises any one selected from SEQ ID NOs: 53 to 66, or a sequence having at least 85% sequence identity thereto, and/or
   the HCDR3 of the antibody comprises any one selected from SEQ ID NOs: 67 to 75, or a sequence having at least 85% sequence identity thereto, and/or
   the LCDR1 of the antibody comprises any one selected from SEQ ID NOs: 76 to 83, or a sequence having at least 85% sequence identity thereto, and/or
   the LCDR2 of the antibody comprises any one selected from SEQ ID NOs: 84 to 90, or a sequence having at least 85% sequence identity thereto, and/or
   the LCDR3 of the antibody comprises any one selected from SEQ ID NOs: 91 to 98, or a sequence having at least 85% sequence identity thereto,
   preferably, the antibody comprises HCDR1, HCDR2, HCDR3 of an antibody shown in any one row of Table 1, or a sequence having at least 85% sequence identity thereto, and/or the antibody comprises LCDR1, LCDR2 and LCDR3 of an antibody shown in any one row of Table 1, or a sequence having at least 85% sequence identity thereto,
   more preferably, the antibody comprises HCDR1, HCDR2, HCDR3, LCDR1, LCDR2 and LCDR3 of the antibody shown in any one row of Table 1.
3. The antibody or antigen-binding fragment thereof according to item 1 or 2, wherein the light chain variable region of the antibody comprises a murine or human light chain FR region, and the heavy chain variable region of the antibody comprises a murine or human heavy chain FR region,
   preferably, FR1, FR2, FR3 and FR4 of the heavy chain variable region of the antibody are each independently selected from FR1, FR2, FR3 and FR4 of the heavy chain variable region shown by any one of SEQ ID NOs: 1 to 23, and/or, FR1, FR2, FR3 and FR4 of the light chain variable region of the antibody are each independently selected from FR1, FR2, FR3 and FR4 of the light chain variable region shown by any one of SEQ ID NOs: 24 to 41,
   more preferably, the antibody comprises VH, VL, or VH and VL of an antibody shown in any one row of Table 1, or a sequence having at least 85% sequence identity thereto.
4. The antibody or antigen-binding fragment thereof according to item 1 or 2, wherein,
   the antibody further comprises a heavy chain constant region and/or a light chain constant region, and/or
   the antibody is a multispecific antibody, and/or
   the antibody is a monoclonal antibody, and/or
   the antibody is a chimeric antibody or a fully human antibody.
5. A fusion protein or antibody conjugate comprising the antibody or antigen-binding fragment thereof according to any one of items 1 to 4.
6. A polynucleotide, selected from:
   (1) the coding sequence of the antibody or antigen-binding fragment thereof according to any one of items 1 to 4, or the fusion protein or antibody conjugate according to item 5;
   (2) a complement sequence of (1).
7. A nucleic acid construct, which expresses the antibody or antigen-binding fragment thereof according to any one of items 1 to 4, or expresses the fusion protein or antibody conjugate according to item 5, or comprises the polynucleotide according to item 6,
   preferably, the nucleic acid construct is a vector.
8. A phage comprising the antibody or antigen-binding fragment thereof according to any one of items 1 to 4, or a library comprising the phage.
9. A host cell, which:
   (1) expressing and/or secreting the antibody or antigen-binding fragment thereof according to any one of items 1 to 4;
   (2) comprising the polynucleotide according to item 6; and/or
   (3) comprising the nucleic acid construct according to item 7,
   preferably, the host cell is a mammalian cell.
10. A method for producing an antibody or an antigen-binding fragment thereof, comprising: culturing the host cell according to item 9 under conditions suitable for producing the antibody or the antigen-binding fragment thereof, and optionally purifying the antibody or the antigen-binding fragment thereof from the culture.
11. A pharmaceutical composition, comprising the antibody or antigen-binding fragment thereof according to any one of items 1 to 4, fusion protein, antibody conjugate, polynucleotide, nucleic acid construct, phage or host cell, and a pharmaceutically acceptable excipient.
12. Use of the antibody or antigen-binding fragment thereof according to any one of items 1 to 4, the fusion protein or antibody conjugate according to item 5, the polynucleotide according to item 6, the nucleic acid construct according to item 7, the bacteriophage according to item 9 or the host cell according to item 9 in the preparation of a medicament for preventing or treating a disease,
   preferably, the diseases include: cancer, endometriosis, alopecia, osteoporosis, obesity, benign lesions caused by PRLR activation,
   more preferably, the cancer is a PRLR-related cancer,
   further preferably, the cancer is selected from the group consisting of: ovarian cancer, melanoma, prostate cancer, intestinal cancer, gastric cancer, esophageal cancer, breast cancer, lung cancer, kidney cancer, pancreatic cancer, uterine cancer, liver cancer, bladder cancer, cervical cancer, oral cancer, brain cancer, testicular cancer, skin cancer, thyroid cancer and hematological malignancies.
13. A kit for detecting PRLR, for evaluating drug treatment effects or diagnosing cancer, wherein the kit comprises the antibody or antigen-binding fragment thereof according to any one of items 1 to 4, the fusion protein or antibody conjugate according to item 5, the polynucleotide according to item 6, the nucleic acid construct according to item 7, the bacteriophage according to item 8, or the host cell according to item 9,
   preferably, the kit further comprises a reagent for detecting the binding of PRLR to an antibody or antigen-binding fragment thereof, fusion protein, or antibody conjugate.
14. Use of the antibody or antigen-binding fragment thereof according to any one of items 1 to 4, the fusion protein or antibody conjugate according to item 5, the polynucleotide according to item 6, the nucleic acid construct according to item 7, the bacteriophage according to item 8 or the host cell according to item 9 in the preparation of a kit for detecting PRLR in a sample, evaluating the efficacy of drug treatment or diagnosing cancer.

The present invention is described in further detail with reference to the following experimental examples. These examples are provided for illustrative purposes only and are not intended to be limiting unless otherwise specified. Accordingly, the present invention should in no way be construed as limited to the following examples, but should be construed to include any and all changes that become apparent in light of the teachings provided herein. Unless otherwise specified, the methods and materials used in the examples are conventional materials and methods in the art.

### EXAMPLE

The present invention is further described below in conjunction with examples, but these examples are not intended to limit the scope of the present invention. Experimental methods in the examples of the present disclosure that do not specify specific conditions are generally performed under conventional conditions, such as the Antibody Technology Laboratory Manual (Cold Spring Harbor Laboratory, CSHL) and the Molecular Cloning Manual; or according to the experimental conditions recommended by the raw material or product manufacturer. Reagents that do not specify specific sources are conventional reagents purchased from the market.

### Example 1. Preparation of PRLR antigen and detection protein

Using the extracellular domain (amino acid sequence at positions 25-234) of human PRLR protein (Uniprot number: P16471) or the extracellular domain (amino acid sequence at positions 25-234) of cynomolgus monkey PRLR protein (Uniprot number: G8F5S4) as templates, antigens and detection proteins with different fusion tags were designed, including avi-(his)6 tag (amino acids 211-231 of SEQ ID NO: 99) and mouse IgG2a Fc (mFc) tag (amino acids 214-446 of SEQ ID NO: 100). The extracellular segment of human PRLR protein with avi-(his)6 tag (hPRLR-ECD-avi-his) is shown by SEQ ID NO: 99. The extracellular segment of human PRLR protein with mouse IgG2a Fc (mFc) tag (hPRLR-ECD-mFc) is shown by SEQ ID NO: 100. The extracellular segment of the PRLR protein of cynomolgus monkey with avi-(his)6 tag (cynoPRLR-ECD-avi-his) is shown by SEQ ID NO:101.

The DNA sequence encoding the above protein was synthesized (Sangon Biotech (Shanghai) Co., Ltd.) and inserted into the pcDNA3.1 expression vector to construct a plasmid expressing the recombinant protein. The target plasmid was transfected into Expi293F (Thermo) cells using PEI (polyscience) to express the protein. The cell culture supernatant was collected after four days, and the cell culture supernatant was collected after high-speed centrifugation and filtered with a 0.22 µm filter to remove residual cell debris.

The antigen protein was purified using a nickel column or a Protein A column (GE) according to the different tags it carried. The purified samples were concentrated, identified by SDS-PAGE and mass spectrometry, and then aliquoted and cryopreserved at -80°C for later use.

### Example 2. Construction of PRLR-expressing cell line

A DNA encoding the complete sequence of human PRLR (Uniprot number: P16471, 1-622) and carrying a P2A-GFP tag sequence (SEQ ID NO: 102, position 625-882) was synthesized (the encoded amino acid sequence is shown by SEQ ID NO: 102) and inserted into the pcDNA3.1 expression vector. The purified plasmid was digested with Pvu I (NEB) and transfected into HEK293, Cos7 or CHO K1 cells using Lipofectamine LTX (Thermo). After 24 hours of transfection, 700-1000 µg/mL of G418 was added for screening. After continuous cultivation in a culture medium containing G418 for 2 weeks, GFP expression was detected by flow cytometry and GFP-positive cells were sorted. Stable cell lines HEK293-PRLR, CHOK1-PRLR and Cos7-PRLR expressing PRLR were constructed by single cell sorting and amplification culture.

### Example 3. Acquisition and preparation of anti-human PRLR hybridoma monoclonal antibodies

### 1. Mouse Immunization

6-8 week old Balb/c female mice (Guangdong Pharmicron Biological Technology Co., Ltd.) were selected , and mixed the recombinant expressed protein antigen hPRLR-ECD-avi-his or hPRLR-ECD-mFc (protein concentration: 1-2 mg/mL) with an equal volume of adjuvant [optional: Freund's complete adjuvant (Sigma, F5881-10ML), Freund's incomplete adjuvant (344291-10ML), TiterMax Gold adjuvant (Sigma, T2684), Imject^{®} Alum (Thermo, 77161), etc.] for immunization. The antigen emulsion prepared with Freund's complete adjuvant or TiterMax Gold adjuvant was used for the first immunization, and 50-100ug antigen was injected subcutaneously per mouse. Booster immunization was performed on the 14th and 28th days after the first immunization, and the antigen suspension was prepared by mixing protein with Freund's incomplete adjuvant or Imject^{®} Alum. 25-50ug antigen was injected intraperitoneally or subcutaneously per mouse. On the 35th day, blood was collected to test the antibody titer. The antibody titer was determined based on whether to continue the booster immunization or the last rush immunization. For rush immunization, 50-100 µg of pure protein solution (protein dissolved in phosphate buffer or saline) was injected through the tail vein of mice. The mice were euthanized 3 days after immunization, and blood, spleen and lymph node samples were collected for detection and hybridoma preparation.

### 2. Hybridoma Preparation

Hybridoma cells were prepared by methods known to those skilled in the art. Specifically, freshly collected mouse spleen cells or lymph node cells were prepared into a single cell suspension. Red blood cells are lysed by adding red blood cell lysis buffer (Beyotime Biotechnology Co., Ltd., C3702) at room temperature for 5 minutes, centrifuged and counted, and then mixed with Sp2/0 myeloma cell line (spleen cell: sp2/0 cell ratio is 2:1), and then washed thoroughly with 20mL electrofusion buffer (BTX cytofusion mediumc C) and electrofused (BTX ECM2001) to prepare hybridoma cells. A 9 mL fusion pool was used, and the electrofusion parameters were set as follows (AC parameters: 75 V/75 V/30 s/1.0 MHz; DC parameters: 800 V/40 µs/1/0.000 s; AC parameters: 75 V/75 V/30 s/1.0 MHz). A volume of 7.2 mL of the fully mixed cell suspension was added to the fusion pool, and fusion was started immediately. The fused hybridoma cells were resuspended in HAT complete medium (RPMI-1640 medium containing 10% FBS, 1×HAT, 1×P/S, 1×Glutamax, and 1×pyruvate), and inoculated in a 96-well cell culture plate (1×10⁵ /100 µl/well), and cultured at 37°C and 5% CO₂. The medium was changed every 3 days. On the 14th day after fusion, ELISA detection was performed according to the cell growth.

### 3. Hybridoma cell screening

A 96-well high adsorption plate (Nunc MaxiSorp^{™}, 44-2404-21) was coated with 3 µg/mL antigen protein (100 µL/well) and incubated overnight at 4°C. The next day, the 96-well plate was blocked with PBS containing 3% skim milk powder (300µL/well) and incubated at room temperature for 1 hour. After washing the 96-well plate 3 times with PBS washing solution containing 0.1% Tween-20, 100 µL of hybridoma supernatant was added to each well and placed at room temperature for 1 hour. The 96-well plate was washed three times, and 100 µL of 2000-fold diluted HRP-labeled goat anti-mouse antibody (Thermo, A10521) was added to each well and incubated at room temperature for 1 hour. After washing the plate 4 times with washing solution, 50 µL TMB colorimetric solution (Invitrogen, 002023) was added per well for color development for 2-5 minutes. 1M sulfuric acid was added to terminate the reaction, and the absorbance at 450nm was recorded with a microplate reader. Positive hybridomas were flow-sorted, seeded at 1 cell/well in a new 96-well plate, then cultured, expanded, and cryopreserved.

### Example 4. Construction and screening of phage display library of anti-human PRLR antibodies

By extracting RNA from spleen cells of immune mice and synthesizing cDNA, a single-chain antibody (scFv)-phage display library was constructed, and the target antibody is screened by phage surface display technology. Specifically, after synthesizing cDNA, PCR amplification was performed using degenerate primers, such as the primer combination given by A Krebber et al., Journal of Immunological Methods 201 (1997) 35-55, to obtain the heavy chain and light chain sequence DNA of the target antibody and constructed a single-chain antibody-phage display immune library. The target clone sequence was obtained by various panning methods known to those skilled in the art.

### Example 5. Anti-PRLR antibody heavy chain and light chain variable region sequences

Table 2 lists the sequence numbers corresponding to the amino acid sequences of the heavy chain, light chain variable region and CDR region of the anti-PRLR antibody obtained in the present invention. The sequences with P as the prefix of the antibody number were from phage display screening, the sequences with H as the prefix were from hybridoma screening, and the sequences with HP as the prefix were from the phage library constructed with the cDNA of positive hybridoma clones.

**Table 2. List of heavy chain and light chain sequences of murine anti-PRLR antibodies**

| | **SEQ ID NOs** | | | | | | | |
|---|---|---|---|---|---|---|---|---|
| **Antibody No.** | **VH** | **HCDR1** | **HCDR2** | **HCDR3** | **VL** | **LCDR1** | **LCDR2** | **LCDR3** |
| P-PR-C02H | 1 | 42 | 53 | 67 | 24 | 76 | 84 | 91 |
| P-PR-C04H | 2 | 42 | 54 | 67 | 25 | 77 | 84 | 92 |
| P-PR-C05H | 3 | 43 | 55 | 67 | 26 | 77 | 84 | 91 |
| P-PR-C06H | 4 | 42 | 53 | 67 | 27 | 77 | 85 | 92 |
| P-PR-C07H | 5 | 44 | 56 | 68 | 28 | 77 | 84 | 91 |
| P-PR-C08H | 6 | 42 | 53 | 67 | 28 | 77 | 84 | 91 |
| P-PR-C12H | 7 | 45 | 57 | 67 | 29 | 77 | 84 | 92 |
| P-PR-D01H | 8 | 42 | 54 | 67 | 28 | 77 | 84 | 91 |
| P-PR-D04H | 9 | 46 | 58 | 69 | 30 | 78 | 86 | 93 |
| P-PR-B09W | 10 | 43 | 59 | 67 | 28 | 77 | 84 | 91 |
| P-PR-G02W | 11 | 42 | 53 | 67 | 28 | 77 | 84 | 91 |
| P-PR-H04W | 4 | 42 | 53 | 67 | 25 | 77 | 84 | 92 |
| P-PR-H07W | 7 | 45 | 57 | 67 | 31 | 77 | 84 | 92 |
| P-PR-H11W | 3 | 43 | 55 | 67 | 31 | 77 | 84 | 92 |
| P-PR-C04W | 6 | 42 | 53 | 67 | 28 | 77 | 84 | 91 |
| P-PR-D06H | 12 | 43 | 55 | 67 | 32 | 79 | 87 | 92 |
| H-PR-B03 | 13 | 47 | 60 | 70 | 33 | 80 | 88 | 94 |
| H-PR-B06 | 14 | 48 | 61 | 71 | 34 | 81 | 89 | 95 |
| HP-PR02E11 | 15 | 49 | 62 | 72 | 35 | 80 | 88 | 96 |
| HP-PR01D12 | 16 | 50 | 63 | 73 | 36 | 82 | 90 | 97 |
| HP-PR02H02 | 17 | 48 | 61 | 71 | 37 | 81 | 89 | 95 |
| HP-PR01C02 | 18 | 51 | 64 | 74 | 38 | 83 | 90 | 98 |
| HP-PR01B01 | 19 | 52 | 65 | 75 | 39 | 83 | 90 | 98 |
| P-PR-C05H-v1 | 20 | 43 | 55 | 67 | 40 | 77 | 84 | 91 |
| P-PR-C05H-v2 | 21 | 43 | 66 | 67 | 40 | 77 | 84 | 91 |
| P-PR-C07H-v | 22 | 44 | 56 | 68 | 40 | 77 | 84 | 91 |
| HP-PR01D12-v | 23 | 50 | 63 | 73 | 41 | 82 | 90 | 97 |

### Example 6. Expression and purification of anti-PRLR antibodies

Primers were designed and PCR amplified to obtain the VH and VL gene fragments of the antibody in Table 2, and overlap PCR was performed with the constant region gene fragments CH1-Hinge-CH2-CH3 and CL of the heavy chain and light chain of human IgG1 to obtain the fusion DNA sequence, which was inserted into the mammalian expression vector pCDNA3.1 to construct the heavy and light chain plasmid vectors expressing chimeric antibodies. After sequencing to verify that the sequence was correct, the plasmid vector was extracted using an endotoxin-free plasmid extraction kit and stored at -20°C for standby use. Expi293F was diluted to a density of approximately 4x10⁶ cells/mL, and the plasmids expressing the light and heavy chain of the antibody were co-transfected into Expi293F cells using PEI40000 (polysciences) transfection reagent. The cell culture supernatant was collected after four days, and the cell culture supernatant was collected after high-speed centrifugation and filtered with a 0.22 µm filter to remove residual cell debris. The filtered cell culture supernatant was purified using a Protein A column, and the Protein A column was rinsed with PBS buffer to remove impurities. After the A280 reading dropped to a stable baseline, the target protein was eluted with a 0.1M acetic acid-sodium acetate solution at pH 3.2, and the target protein peak was collected and neutralized with a 1M Tris-HCl, pH 8.0 solution. After the sample was concentrated, it was further purified using a gel chromatography column ENrichTM SEC650 (Bio-red) to remove aggregates and collect the monomer peak. The collected samples were detected by 4-12% SDS-PAGE gradient gel electrophoresis, and then aliquoted and stored at -80°C for later use.

### Example 7. Anti-PRLR antibody species-specific ELISA detection

The monomeric mouse, monkey and human PRLR extracellular domain recombinant antigen protein was diluted into PBS buffer (final concentration of 1 µg/mL), and the antigen protein was added to a 96-well high adsorption plate (100 µL/well) and incubated at 4°C overnight. The next day, the the 96-well plate was blocked with PBS containing 3% skim milk powder (300µL/well) and placed at room temperature for 1 hour. The 96-well plate was washed three times with PBS washing solution containing 0.1% Tween-20, 100 µL of purified antibody to be tested (10 µg/mL diluted in PBS) was added to each well and placed at room temperature for 1 hour. Wash the 96-well plate 3 times, add 100 µL of 2000-fold diluted HRP-labeled goat anti-mouse antibody (Thermo) to each well, and incubate at room temperature for 1 hour. After washing the plate 4 times with washing solution, 50 µL TMB colorimetric solution (Invitrogen, 002023) was added per well for color development for 2-5 minutes. 1M sulfuric acid was added to terminate the reaction, and the absorbance at 450nm was recorded with a microplate reader. Table 3 shows the species-specific ELISA test results of the antibodies obtained by the present invention, and the control protein is a recombinant mesothelin protein with the same tag. As shown in Table 3, the obtained antibodies all specifically bind to the extracellular segment of human PRLR, and because the extracellular segment of cynomolgus monkey PRLR has a high sequence consistency with the extracellular segment of human PRLR, only 5 amino acid sites are different (sequence consistency: 97.6%), among the 23 antibody strains, except for antibodies No. 18, 21 and 23, most of the antibodies can bind to the extracellular segment of cynomolgus monkey PRLR. In addition, antibodies No. 3, 7, 9, 16 and 19 can also bind to the extracellular segment of mouse PRLR.

**Table 3. Species-specific detection of anti-PRLR monoclonal antibodies**

| Antibody No. | Antibody Name | Control protein | human PRLR extracellular domain | murine PRLR extracellular domain | cynomolgus monkey PRLR extracellular domain |
|---|---|---|---|---|---|
| 1 | P-PR-C02H | 0.098 | 2.138 | 0.115 | 2.302 |
| 2 | P-PR-C04H | 0.09 | 2.152 | 0.978 | 2.397 |
| 3 | P-PR-C05H | 0.116 | 2.141 | 2.082 | 2.324 |
| 4 | P-PR-C06H | 0.084 | 2.101 | 0.102 | 2.281 |
| 5 | P-PR-C07H | 0.062 | 2.286 | 0.102 | 2.162 |
| 6 | P-PR-C08H | 0.065 | 2.199 | 0.094 | 2.255 |
| 7 | P-PR-C12H | 0.091 | 2.242 | 1.128 | 2.313 |
| 8 | P-PR-D01H | 0.068 | 2.015 | 0.118 | 2.368 |
| 9 | P-PR-D04H | 0.065 | 1.755 | 1.861 | 2.096 |
| 10 | P-PR-B09W | 0.102 | 2.32 | 0.141 | 2.514 |
| 11 | P-PR-G02W | 0.07 | 2.248 | 0.09 | 2.514 |
| 12 | P-PR-H04W | 0.064 | 0.865 | 0.086 | 0.804 |
| 13 | P-PR-H07W | 0.069 | 2.45 | 0.23 | 2.22 |
| 14 | P-PR-H11W | 0.054 | 2.317 | 0.471 | 2.458 |
| 15 | P-PR-C04W | 0.095 | 2.16 | 0.09 | 2.25 |
| 16 | P-PR-D06W | 0.137 | 2.484 | 2.515 | 2.514 |
| 17 | H-PR-B03 | 0.067 | 4 | 0.382 | 3.753 |
| 18 | H-PR-B06 | 0.054 | 3.942 | 0.334 | 0.435 |
| 19 | HP-PR02E11 | 0.111 | 3.908 | 3.441 | 3.645 |
| 20 | HP-PR01D12 | 0.09 | 2.017 | 0.139 | 3.049 |
| 21 | HP-PR02H02 | 0.076 | 3.122 | 0.223 | 0.097 |
| 22 | HP-PR01C02 | 0.072 | 1.448 | 0.273 | 1.516 |
| 23 | HP-PR01B01 | 0.076 | 0.638 | 0.161 | 0.077 |

### Example 7. Affinity detection of anti-PRLR antibodies

Biomembrane interferometry (OCTET R2, Sartorius) was used to analyze the kinetic parameters (kon, koff and KD) of antibody binding to PRLR ECD. The protein A sensor (Octet^{®} ProA Biosensors) was immersed in a PBS buffer containing 0.1% BSA, 0.05% Tween20, pH 7.4 containing the antibody to be analyzed (200nM) to allow the antibody to bind to the sensor surface. After washing, the sensor was immersed in a buffer solution containing different concentrations of monomeric PRLR antigen in turn, and the affinity parameter KD was obtained by fitting the 1:1 Langmuir model. It can be seen from Table 4 that most antibodies can bind to the target antigen with high affinity, and the binding affinity is between nM and pM.

**Table 4. Kinetic parameters of binding of anti-PRLR monoclonal antibodies to the extracellular domain of monomeric PRLR**

| Antibody No. | Antibody Name | kon (M⁻¹s⁻¹) | koff (s⁻¹) | KD (M) |
|---|---|---|---|---|
| 1 | P-PR-C02H | 1.53E+05 | 2.44E-07 | 1.60E-12 |
| 2 | P-PR-C04H | 2.48E+05 | 2.44E-07 | <1.0E-12 |
| 3 | P-PR-C05H | 2.09E+05 | 1.30E-07 | <1.0E-12 |
| 4 | P-PR-C06H | 2.44E+05 | 6.63E-04 | 2.71E-09 |
| 5 | P-PR-C07H | 1.06E+05 | 3.86E-04 | 3.64E-09 |
| 6 | P-PR-C08H | 1.38E+05 | 2.44E-07 | 1.77E-12 |
| 7 | P-PR-C12H | 1.22E+05 | 3.30E-04 | 2.70E-09 |
| 8 | P-PR-D01H | 2.32E+05 | 2.44E-07 | 1.05E-12 |
| 9 | P-PR-B09W | 1.87E+05 | 2.87E-04 | 1.53E-09 |
| 10 | P-PR-H07W | 1.76E+05 | 9.15E-04 | 5.20E-09 |
| 11 | P-PR-H11W | 1.30E+05 | 2.92E-04 | 2.25E-09 |
| 12 | P-PR-D06H | 1.15E+05 | 3.89E-04 | 3.38E-09 |

### Example 10 Humanization of anti-human PRLR monoclonal antibody

The humanized antibody sequence with high homology to the target murine antibody sequence is selected as a template through sequence alignment. The CDR region of the mouse antibody is determined by the IMGT numbering system, and the CDR region sequences of the heavy chain and light chain are transplanted to the humanized template respectively, while retaining the amino acids in the following structural positions as murine amino acids, such as Vernier Zone amino acids (Foote et al. J. Mol. Biol, 1992, 224, 487-499); amino acids at the interface of the heavy and light chains and showing an effect on the pairing of the heavy and light chains in the structural model; amino acids that affect the structure of the antibody CDR region (Vargas-Madrazo et al. J. Mol. Biol, 1995, 254, 497-504; Al-Lazikani et al. J. Mol. Biol, 1997, 273, 927-948), thereby obtaining a humanized sequence. 1. Humanization of clone P-PR-C05H

The human antibody common sequence was used as the heavy chain and light chain template, the murine CDR region sequence was grafted onto the human template, and the amino acid sites critical to the structure and function were retained as murine amino acids (as shown in Table 5), and the P-PR-C05H-v1 heavy chain (SEQ ID NO: 20) and light chain sequence (SEQ ID NO: 40) were obtained. The P-PR-C05H-v1 heavy chain sequence was mutated to S57A to obtain the humanized antibody P-PR-C05H-v2 (heavy chain sequence: SEQ ID NO: 21), and the antibody sequence was numbered according to the IMGT numbering rules.

**Table 5. Key murine amino acid sites retained in humanization of P-PR-C05H**

| sequence name | sequence type | Retained murine amino acids and positions |
|---|---|---|
| | VH:FR2 | V39,I53,N55 |
| P-PR-C05H-v1 | VH:FR3 | A76,L78,V80 |
| | VL:FR2 | Y40,F42 |

### 2. Humanization of clone P-PR-C07H

The human antibody common sequence was used as the heavy chain and light chain template, the murine CDR region sequence was grafted onto the human template, and the amino acid sites critical to the structure and function were retained as murine amino acids (as shown in Table 6), and the P-PR-C07H-v1 heavy chain (SEQ ID NO: 22) and light chain sequence (SEQ ID NO: 40) were obtained.

**Table 6. Key murine amino acid sites retained in humanization of P-PR-C07H**

| sequence name | sequence type | Retained murine amino acids and positions |
|---|---|---|
| | VH: FR2 | L39,A40,I42,I53,D55 |
| P-PR-C07H | VH: FR3 | N68,L78,F103 |
| | VL: FR2 | Y40,F42 |

### 3.Humanization of cloning HP-PR01 D12

The human antibody common sequence was used as the heavy chain and light chain template, the murine CDR region sequence was grafted onto the human template, and the amino acid sites critical to the structure and function were retained as murine amino acids (as shown in Table 7), and the P-PR-C07H-v1 heavy chain (SEQ ID NO: 23) and light chain sequence (SEQ ID NO: 41) were obtained.

**Table 7. Key murine amino acid sites retained in humanization of HP-PR01 D12**

| sequence name | sequence type | Retained murine amino acids and positions |
|---|---|---|
| | VH:FR2 | N40,A54,Y55 |
| HP-PR01D12 | VL:FR2 | H40,W53 |
| | VL:FR3 | K66,A68,N85,Y87 |

### Example 11. Affinity detection and epitope analysis of humanized PRLR-specific antibodies

The monomeric human or cynomolgus monkey PRLR ECD protein was biotin labeled, and the labeled protein was diluted in (100nM) PBS buffer containing 0.1% BSA, 0.05% Tween20, pH 7.4. Then the streptavidin sensor was immersed in the solution containing the biotin labeled protein to solidify the target antigen. After washing the sensor with the buffer, the sensor was immersed in the buffer containing different concentrations (3.125-100nM) of humanized antibodies in turn, binding for 2 minutes and dissociation for 8 minutes. The kinetic curves of the binding of humanized antibodies labeled as P-PR-C05H-v2, P-PR-C07H-v and HP-PR01 D12-v to human PRLR or monkey PRLR ECD were obtained after subtracting the control sensor. The affinity parameter KD was obtained by fitting the 1:1 Langmuir model. As shown in Table 8, the three humanized antibodies can bind to human and monkey PRLR ECD proteins with high affinity.

**Table 8. Summary of kinetic and affinity data of PRLR antibodies**

| **Antibody** | | **Human PRLR** | | | **Monkey PRLR** | |
|---|---|---|---|---|---|---|
| | **KD (M)** | **kon (M⁻¹s⁻¹)** | **koff (s⁻¹)** | **KD (M)** | **kon (M⁻¹s⁻¹)** | **Koff (s⁻¹)** |
| P-PR-C05H-v2 | <1.0E-12 | 9.37E+05 | 5.03E-07 | <1.0E-12 | 1.04E+06 | 4.08E-07 |
| hP-PR-C07H-v | 3.94E-09 | 8.13E+05 | 3.20E-03 | <1.0E-12 | 6.63E+05 | 4.19E-07 |
| hHP-PR01D12-v | 1.13E-12 | 4.31E+05 | 4.88E-07 | <1.0E-12 | 4.53E+05 | 3.43E-07 |

The antigen binding epitopes of three humanized antibodies were analyzed by BLI. The human monomer PRLR ECD with a biotin tag was fixed to a streptavidin (SA) sensor. Anti-P-PR-C05H-v2, hP-PR-C07H-v and HP-PR01D12-v antibodies were diluted to a final concentration of 100nM with PBS buffer containing 0.1% BSA, 0.05% Tween20, pH 7.4, and the sensor was immersed in the first antibody sample until the signal reached saturation, and then the sensor was immersed in 100nM of the second antibody. The first and second antibodies that recognize the same epitope, under the premise that the first antibody saturates the epitope on the surface of PRLR, the second antibody cannot bind to PRLR, and when there are overlapping epitopes, the binding signal of the second antibody is reduced. For different epitopes, the binding signal of the second antibody is basically consistent with the reference (i.e., the first antibody is a buffer). The distribution of epitopes is analyzed by the ratio of the binding signal of the second antibody to the reference. Where the ratio is less than 20%, the two antibodies have the same epitope, between 20%-60%, there is partial overlap in the epitopes, and >60%, the epitopes do not overlap at all. The epitopes of the three humanized antibodies are shown in Table 9. It can be seen that P-PR-C05H-v2 and HP-PR01D12-v bind to the same epitope of the antigen, while the antigen binding epitopes of P-PR-C07H-v are close to P-PR-C05H-v2 and HP-PR01 D12-v, but do not completely overlap.

**Table 9. Epitope analysis of humanized antibodies binding to human PRLR**

| | P-PR-C05H-v2 | P-PR-C07H-v | HP-PR01D12-v |
|---|---|---|---|
| P-PR-C05H-v2 | 11.2% | 35.0% | 15.5% |
| P-PR-C07H-v | 52.7% | 13.4% | 49.8% |
| HP-PR01 D12-v | 9.2% | 31.8% | 14.4% |

### Example 12. Humanized PRLR specific antibody mediated receptor internalization detection

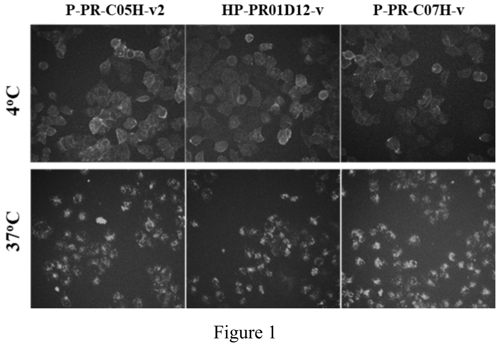

The internalization of humanized antibodies P-PR-C05H-v2, P-PR-C07H-v and HP-PR01 D12-v after binding to PRLR was detected by immunofluorescence. After incubating 10 µg/mL of humanized antibodies with T47D cell line (breast cancer cell line endogenously expressing human PRLR) at 4°C for 1 hour, the excess antibodies were washed away, and anti-human Fc antibodies labeled with FITC fluorescence were added, and the cells were incubated at 4°C for another 1 hour. The excess antibodies were washed away, and fresh culture medium was added. The cells of the control group were placed at 4°C, and the cells of the internalization group were placed at physiological temperature (37°C, carbon dioxide incubator). After 1 hour, the cells were removed, fixed with 4% paraformaldehyde, and the internalization of cell surface antigens was observed under a fluorescence microscope. As shown in Figure 1, the antibodies internalized into the cells can be clearly observed after only 1 hour of incubation at 37°C.

### Example 13. Humanized PRLR-specific antibodies block PRL-mediated PRLR activation and signal transduction

The luciferase reporter gene method was used to determine whether humanized PRLR-specific antibodies blocked prolactin (PRL)-mediated receptor dimer conformational changes and downstream signal transduction. Specifically, the plasmid pGL4.52 (Promega, #E465A) encoding the STAT5-dependent luciferase reporter gene was transfected into HEK293 cells stably expressing PRLR by Lipofectamine LTX (Thermo, #15338-100). After 24 hours of transfection, the cells were digested with trypsin, counted, and inoculated into 96-well white plates at a ratio of 5x10⁴ cells/well, and then a constant concentration of 5nM PRL was mixed with different concentrations of humanized antibodies (50pM to 100nM) and added to the cells. And the cells were incubated at 37°C for 5 hours. After the incubation, the activity of luciferase in each well was determined using Bright-GloTM reagent (Promega, #E2610). The IC50 value that humanized antibody blocks PRL-mediated receptor activation was calculated. As shown in Table 10, P-PR-C05H-v2, P-PR-C07H-v and HP-PR01 D12-v have different abilities of blocking PRL-mediated receptor activation, wherein P-PR-C07H-v can completely block PRL-mediated receptor activation (signal is baseline value) under the concentration condition of 100nM. In addition, P-PR-C05H-v2, P-PR-C07H-v and HP-PR01D12-v themselves do not activate PRLR under the test concentration of (50pM to 100nM).

**Table 10. Humanized antibodies block PRL-mediated receptor activation**

| | IC50 for blocking 5 nM PRL | Blocking percentage (%) of 100 nM antibody |
|---|---|---|
| P-PR-C05H-v2 | 80.39 | 17.3 |
| P-PR-C07H-v | 1.95 | 100.0 |
| HP-PR01D12-v | 23.17 | 46.7 |

### Example 14. Detection of target cell killing mediated by anti-PRLR antibody-DT3C

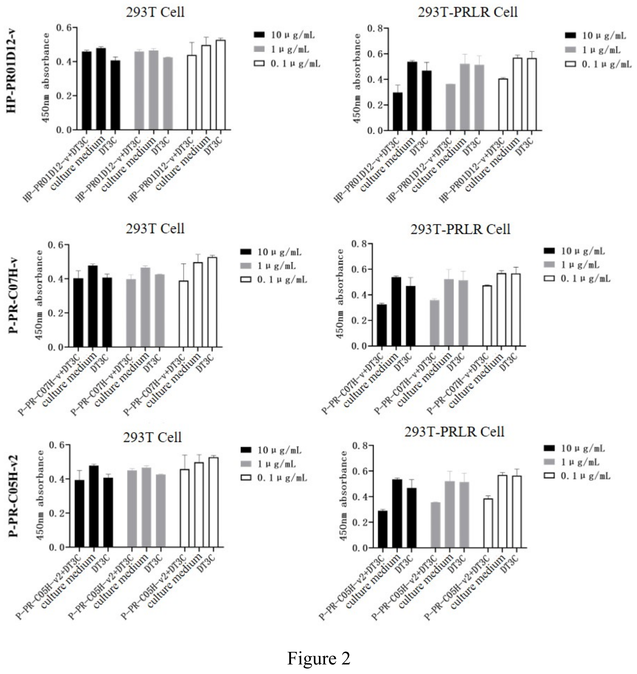

293T cells and 293T-PRLR cells (5000 cells/100 µL/well) were inoculated into 96-well cell culture plates. After 24 hours of culture, anti-PRLR antibodies and DT3C proteins were added to fresh culture medium and incubated at 37°C. The final concentrations of anti-PRLR antibodies were 0.1, 1, and 10 µg/mL, and the corresponding DT3C concentrations were 0.2, 2, and 20 µg/mL. After the incubation, the culture medium in the original 96-well plate was discarded, and the samples to be tested were added to the cells. A culture medium control group and a DT3C control group were set up at the same time. The cells were cultured for 24 hours at 37°C. After the culture was completed, 10 µL of CCK8 solution (purchased from TransGen Biotech, catalog number FC101-02) was added to each well. The 96-well plate was wrapped with tin foil and incubated in a 37°C incubator for 1 hour, and then read using a Synergy H1 microplate reader (purchased from BioTek) to measure the absorbance at 450 nm. As shown in Figure 2, all three humanized antibodies could effectively mediate DT3C's killing of PRLR-positive target cells but not cells that did not express PRLR.

### The sequences of the present invention

| SEQ ID NO: | Sequences |
|---|---|
| 1 | QVQLQQPGSELVRPGASVRLSCKASGFTFTTYWVHWVKQRPGQGLEWIGNISPGSGSTNYDEKFKSKASLTV DTFSNTAYMQLSSLTSEDSAVYYCIRHSGNYFNAMDYWGQGTSVTVSS |
| 2 | QVQLQQPGSELVRPGASVKLSCKASGFTFTTYWVHWVKQRPGQGLEWIGNISPGGGSTNYDEKFKSKASLT VDTFSNTAYMQLSSLTSEDSAVYYCIRHSGNYFNAMDYWGQGTTVTVSS |
| 3 | QVQLQQPGSELVRPGASVKLSCKASGFTFTTFWVHWVKQRPGQGLEWIGNISPGSTSTNYNEKFKNKASLTV DTFSNTAYMQLSSLTSEDSAVYYCIRHSGNYFNAMDYWGQGTSVTVSS |
| 4 | QVQLQQPGSELVRPGASVKLSCKASGFTFTTYWVHWVKQRPGQGLEWIGNISPGSGSTNYDEKFKSKASLTV DTFSNTAYMQLSSLTSEDSAVYYCIRHSGNYFNAMDYWGQGTSVTVSS |
| 5 | EVQLQQSGAELVRPGTSVQISCKASGYTFTNNWLAWIKQRPGHGLEWIGDIYPGGVYTNYNEKFKGKATLTAD TSSSTAYMQLSSLTSEDSAVYFCYYGDFWSAYWGQGTLVTVSA |
| 6 | QVQLQQPGSELVRPGASVKLSCKASGFTFTTYWVHWVKQRPGQGLEWIGNISPGSGSTNYDEKFKSKASLTV DTFSNTAYMQLSSLTSEDSAVYYCIRHSGNYFNAMDYWGQGTTVTVSS |
| 7 | QVQLQQPGSELVRPGASVKLSCKASGFTFATYWVHWVKQRPGQGLEWIGNISPGSVSTNYDEKFKSKASLTV DTFSNTAYMQLSSLTSEDSAVYYCIRHSGNYFNAMDYWGQGTTLTVSS |
| 8 | QVQLQQPGSELVRPGASVKLSCKASGFTFTTYWVHWVKQRPGQGLEWIGNISPGGGSTNYDEKFKSKASLT VDTFSNTAYMQLGSLTSEDSAVYYCIRHSGNYFNAMDYWGQGTTLTVSS |
| 9 | QVQLQQPGSELVRPGASVKLSCKASGYTFTSYWMHWVKQRPGQGLEWIGNIYPGSGSTNYDEKFKSKATLT VDTSSSTAYMQLSSLTSEDSAVYYCTRGNYRYYFDYWGQGTTLTVSS |
| 10 | QVQLQQPGSELVRPGASVKLSCKASGFTFTTFWVHWVKQRPGQGLEWIGNISPGGTSTNYDEKFKSKASLTV DTFSNTAYMQLSSLTSEDSAVYYCIRHSGNYFNAMDYWGQGTSVTVSS |
| 11 | QVQLQQPGSELVRPGASVKLSCKASGFTFTTYWVHWVKQRPGQGLEWIGNISPGSGSTNYDEKFKSKASLTV DTFSNTAYMQLSSLTSEDSAVYYCIRHSGNYFNAMDYWGQGTTLTVSS |
| 12 | QVQLQQPGSELVRPGASVKLSCKASGFTFTTFWVHWVKQRPGQGLEWIGNISPGSTSTNYNEKFKNKASLTV DTFSNTAYMQLSSLTSEDSAVYYCIRHSGNYFNAMDYWGQGTTLTVSS |
| 13 | QVQLKESGPGLVAPSQSLSITCTVSGFSLTGFGVNWFRQPPGRGLEWLGMIWGDGSTDYNSALKSRLSISKD NSKSQVFLKMNSLQTDDTARYYCARVLYYYGGNSFAYWGQGTLVTVSA |
| 14 | DVKLVESGGGLVKPGGSLKLACAASGFTFSSYTMSWVRQTPEKRLEWVATISSGGSYTYYPDSVKGRFTISR DNAKNTLYLQLSSLKSEDTAMYYCTRDRYYSTTPDWYFDVWGAGTTVTVSS |
| 15 | QVQLQQSGPGLVAPSQSLSITCTVSGFSLTSYGVNWVRQPPGKGLEWLGIIGAGGSTNYNSALMSRLSISKDN SKSQVFLKMNSLQTDDTAMYYCARSLYYYGSSSFAYWGQGTLVTVSA |
| 16 | EVMLVESGGGLVQPGGSRKLSCAASGFTFSSFGMNWVRQAPEKGLEWVAYISSGSSIIYYADTVKGRFTISRD NPKNTLFLQMTSLRSEDTAMYYCTRFYGYIPFDYWGQGTTLTVSS |
| 17 | EVMLVESGGGLVKPGGSLKLACAASGFTFSSYTMSWVRQTPEKRLEWVATISSGGSYTYYPDSVKGRFTISR DNAKNTLYLQLSSLKSEDTAMYYCTRDRYYSTTPDWYFDVWGAGTTLTVSS |
| 18 | DVHLVESGPGLVKPSQSLSLTCTVTGYSITSDYAWNWIRQFPGNKLEWMGYISYSGSTGYSPSLKSRVSITRD TSKNQFFLQLNSVTTEDTATYFCARNNWAFDFWGQGTTLTVSS |
| 19 | EVHLVESGPDLVKPSQSLSLTCTVTGYSITSGYNWHWIRQFPGNKLEWMGYIHYSGSTKYDPSLKSRISFTRD TSRNQFFLQLNSVTTEDRATYYCARSEAGGYSWFAYWGQGTLVTVSA |
| 20 | EVQLVQSGAEVKKPGASVKVSCKASGFTFTTFWVHWVRQAPGQGLEWIGNISPGSTSTNYAQKFQGRATLTV DTSISTAYMELSSLRSEDTAVYYCIRHSGNYFNAMDYWGQGTLVTVSS |
| 21 | EVQLVQSGAEVKKPGASVKVSCKASGFTFTTFWVHWVRQAPGQGLEWIGNIAPGSTSTNYAQKFQGRATLTV DTSISTAYMELSSLRSEDTAVYYCIRHSGNYFNAMDYWGQGTLVTVSS |
| 22 | EVQLVQSGAEVKKPGSSVKVSCKASGYTFTNNWLAWIRQAPGQGLEWIGDIYPGGVYTNYNQKFQGRATLTA DTSTSTAYMELSSLRSEDTAVYFCYYGDFWSAYWGQGTLVTVSS |
| 23 | EVQLVESGGGLVQPGGSLRLSCAASGFTFSSFGMNWVRQAPGKGLEWVAYISSGSSIIYYADSVKGRFTISRD NSKNTLYLQMNSLRAEDTAVYYCTRFYGYIPFDYWGQGTLVTVSS |
| 24 | DIVMTQATPSVPVAPGESVSISCRSSKSLLHTNGYTYLYWFLQRPGQSPQLLIYRMSNLASGVPDRFSGSGSG TAFTLRISRVEAEDVGVYYCMQHLEYPLTFGAGTKLELK |
| 25 | DIVMTQAAPSVPVTPGESVSISCRSSKSLLHSNGNTYLYWFLQRPGQSPQLLIYRMSNLASGVPDRFSGSGSG TAFTLRISRVEAEDVGVYYCMQHLEYPFTFGSGTKLEIK |
| 26 | DIVMTQAAPSVPVTPGESVSISCRSSKSLLHSNGNTYLYWFLQRPGQSPQLLIYRMSNLASGVPDRFSGSGSG TAFTLRISRVEAEDVGIYYCMQHLEYPLTFGAGTKLELK |
| 27 | DIVMTQAAPSVFVTPGESVSISCRSSKSLLHSNGNTYLYWFLQRPGQSPQLLIYQMSNRASGVPDRLSGSGSG TAFTLRISRVEAEDVGVYYCMQHLEYPFTFGGGTKLEIK |
| 28 | DIVMTQAAPSVPVTPGESVSISCRSSKSLLHSNGNTYLYWFLQRPGQSPQLLIYRMSNLASGVPDRFSGSGSG TAFTLRISRVEAEDVGVYYCMQHLEYPLTFGAGTKLELK |
| 29 | DIVMTQAAPSVLVTPGESVSISCRSSKSLLHSNGNTYLYWFLQRPGQSPQLVIYRMSNLASGVPDRFSGSGSG TAFTLRISRVEAEDVGVYYCMQHLEYPFTFGAGTKLELK |
| 30 | DIVLTQSPASLAVSLGQRATISCRASESVDKSGFSFMNWFQQKPGQPPKLLIYAASNRGSGVPARFSGSGSGT DFSLNIHPMEEDDTAMYFCQQSKEVPWTFGGGTKLEIK |
| 31 | DIVMTQAAPSVPVTPGESVSISCRSSKSLLHSNGNTYLYWFLQRPGQSPQLLIYRMSNLASGVPDRFSGSGSG TAFTLRISRVEAEDVGVYYCMQHLEYPFTFGGGTKLEIK |
| 32 | DIVMTQAAPSVPVTPGESVTISCRSSKSLLHSNGKTYLYWFLQRPGQSPQLLIYWMSNLASGVPDRFSGSGSG TAFTLRISRVEAEDVGVYYCMQHLEYPFTFGVGTKLELK |
| 33 | DVLMTQTPLSLPVSLGDQASISCRSSQSIVHSNGNTYLEWYLQKPGQSPKLLIYKVSSRFSGVPDRFSGSGSG TDFTLKISRVEAEDLGVYYCFQGSHVPYTFGGGTKLEIK |
| 34 | DIVLTQSPATLSVTPGDSVSLSCRATQIIFNNLHWYQQKSHESPRLLITYTSQSISGIPSRFSGSGSGTDFTLTIN SVETEDFGMYFCQQSNTWPRTFGGGTKLEIK |
| 35 | DIVMTQTPLSLPVSLGDQASISCRSSQSIVHSNGNTYLEWYLQKPGQSPKLLIYKVSNRFSGVPDRFSGSGSGT DFTLKISRVEAEDLGVYYCFQGSHVPWTFGGGTKLELK |
| 36 | DIVMTQSPAIMSASPGEKVTMTCSASSSLIYMHWYQQKSSTSPKLWIYDTSKLASGVPGRFSGSGSGNSYSLT TSSMEAEDVATYYCFQGSGYPLTFGGGTKLEIK |
| 37 | DIVITQSPATLSVTPGDSVSLSCRATQIIFNNLHWYQQKSHESPRLLITYTSQSISGIPSRFSGSGSGTDFTLTINS VETEDFGMYFCQQSNTWPRTFGGGTKLEIK |
| 38 | DIVLTQSPAIMSASPGEKVTMTCSASSSVNYMHWYQQKSGTSPKRWIYDTSKLASGVPARFSGSGSGTSYSL TISSMEAEDAATYYCQQWSSNPLTFGAGTKLELK |
| 39 | DIVMTQSPAIMSASPGEKVTMACSASSSVNYMHWYQQKSGTSPKRWIYDTSKLASGVPARFSGSGSGTSYSL TISSMEAEDAATYYCQQWSSNPLTFGAGTKLELK |
| 40 | DIVMTQSPLSLPVTPGEPASISCRSSKSLLHSNGNTYLYWFLQKPGQSPQLLIYRMSNRASGVPDRFSGSGSG TDFTLKISRVEAEDVGVYYCMQHLEYPLTFGQGTKVEIK |
| 41 | DIQMTQSPSSLSASVGDRVTITCRASSSLIYLHWYQQKPGKAPKLWIYDTSKLASGVPSRFSGSGSGNDYTLTI SSLQPEDFATYYCFQGSGYPLTFGQGTKVEIK |
| 42 | GFTFTTYW |
| 43 | GFTFTTFW |
| 44 | GYTFTNNW |
| 45 | GFTFATYW |
| 46 | GYTFTSYW |
| 47 | GFSLTGFG |
| 48 | GFTFSSYT |
| 49 | GFSLTSYG |
| 50 | GFTFSSFG |
| 51 | GYSITSDYA |
| 52 | GYSITSGYN |
| 53 | ISPGSGST |
| 54 | ISPGGGST |
| 55 | ISPGSTST |
| 56 | IYPGGVYT |
| 57 | ISPGSVST |
| 58 | IYPGSGST |
| 59 | ISPGGTST |
| 60 | IWGDGST |
| 61 | ISSGGSYT |
| 62 | IGAGGST |
| 63 | ISSGSSII |
| 64 | ISYSGST |
| 65 | IHYSGST |
| 66 | IAPGSTST |
| 67 | IRHSGNYFNAMDY |
| 68 | YYGDFWSAY |
| 69 | TRGNYRYYFDY |
| 70 | ARVLYYYGGNSFAY |
| 71 | TRDRYYSTTPDWYFDV |
| 72 | ARSLYYYGSSSFAY |
| 73 | TRFYGYIPFDY |
| 74 | ARNNWAFDF |
| 75 | ARSEAGGYSWFAY |
| 76 | KSLLHTNGYTY |
| 77 | KSLLHSNGNTY |
| 78 | ESVDKSGFSF |
| 79 | KSLLHSNGKTY |
| 80 | QSIVHSNGNTY |
| 81 | QIIFNN |
| 82 | SSLIY |
| 83 | SSVNY |
| 84 | RMS |
| 85 | QMS |
| 86 | AAS |
| 87 | WMS |
| 88 | KVS |
| 89 | YTS |
| 90 | DTS |
| 91 | MQHLEYPLT |
| 92 | MQHLEYPFT |
| 93 | QQSKEVPWT |
| 94 | FQGSHVPYT |
| 95 | QQSNTWPRT |
| 96 | FQGSHVPWT |
| 97 | FQGSGYPLT |
| 98 | QQWSSNPLT |
| 99 | QLPPGKPEIFKCRSPNKETFTCWWRPGTDGGLPTNYSLTYHREGETLMHECPDYITGGPNSCHFGKQYTSM WRTYIMMVNATNQMGSSFSDELYVDVTYIVQPDPPLELAVEVKQPEDRKPYLWIKWSPPTLIDLKTGWFTLLY EIRLKPEKAAEWEIHFAGQQTEFKILSLHPGQKYLVQVRCKPDHGYWSAWSPATFIQIPSDFTMNDGLNDIFEA QKIEWHEHHHHHH |
| 100 | QLPPGKPEIFKCRSPNKETFTCWWRPGTDGGLPTNYSLTYHREGETLMHECPDYITGGPNSCHFGKQYTSM WRTYIMMVNATNQMGSSFSDELYVDVTYIVQPDPPLELAVEVKQPEDRKPYLWIKWSPPTLIDLKTGWFTLLY EIRLKPEKAAEWEIHFAGQQTEFKILSLHPGQKYLVQVRCKPDHGYWSAWSPATFIQIPSDFTMNDGSGEPRG PTIKPCPPCKCPAPNLLGGPSVFIFPPKIKDVLMISLSPIVTCVVVDVSEDDPDVQISWFVNNVEVHTAQTQTHR EDYNSTLRVVSALPIQHQDWMSGKEFKCKVNNKDLPAPIERTISKPKGSVRAPQVYVLPPPEEEMTKKQVTLT CMVTDFMPEDIYVEWTNNGKTELNYKNTEPVLDSDGSYFMYSKLRVEKKNWVERNSYSCSVVHEGLHNHHT TKSFSRTPGK |
| 101 | QLPPGKPEIFKCRSPNKETFTCWWRPGTDGGLPTNYSLTYHREGETLMHECPDYITGGPNSCHFGKQYTSM WRTYVMMVNATNQMGSSFSDELYVDVTYIVQPDPPLELTVEVKQPEDRKPYLWMKWSPPTLIDLKTGWFTLL |
| | YEIRLKPEKAAEWETHFAGQQTEFKILSLHPGQKYLVQVRCKPDHGYWSAWSPATFIQIPSDFIMNDGLNDIFE AQKIEWHEHHHHHH |
| 102 | MKENVASATVFTLLLFLNTCLLNGQLPPGKPEIFKCRSPNKETFTCWWRPGTDGGLPTNYSLTYHREGETLMH ECPDYITGGPNSCHFGKQYTSMWRTYIMMVNATNQMGSSFSDELYVDVTYIVQPDPPLELAVEVKQPEDRKP YLWIKWSPPTLIDLKTGWFTLLYEIRLKPEKAAEWEIHFAGQQTEFKILSLHPGQKYLVQVRCKPDHGYWSAWS PATFIQIPSDFTMNDTTVWISVAVLSAVICLIIVWAVALKGYSMVTCIFPPVPGPKIKGFDAHLLEKGKSEELLSAL GCQDFPPTSDYEDLLVEYLEVDDSEDQHLMSVHSKEHPSQGMKPTYLDPDTDSGRGSCDSPSLLSEKCEEP QANPSTFYDPEVIEKPENPETTHTWDPQCISMEGKIPYFHAGGSKCSTWPLPQPSQHNPRSSYHNITDVCELA VGPAGAPATLLNEAGKDALKSSQTIKSREEGKATQQREVESFHSETDQDTPWLLPQEKTPFGSAKPLDYVEIH KVNKDGALSLLPKQRENSGKPKKPGTPENNKEYAKVSGVMDNNILVLVPDPHAKNVACFEESAKEAPPSLEQ NQAEKALANFTATSSKCRLQLGGLDYLDPACFTHSFHVDATNFSLLKQAGDVEENPGPMVSKGEELFTGVVPI LVELDGDVNGHKFSVSGEGEGDATYGKLTLKFICTTGKLPVPWPTLVTTLTYGVQCFSRYPDHMKQHDFFKSA MPEGYVQERTIFFKDDGNYKTRAEVKFEGDTLVNRIELKGIDFKEDGNILGHKLEYNYNSHNVYIMADKQKNGI KVNFKIRHNIEDGSVQLADHYQQNTPIGDGPVLLPDNHYLSTQSALSKDPNEKRDHMVLLEFVTAAGITLGMDE LYK |

## Claims

1. An antibody or an antigen-binding fragment thereof targeting PRLR, or a variant having at least 85% sequence identity with the antibody or antigen-binding fragment thereof and retaining the PRLR binding activity, wherein the antibody targets the extracellular domain of the prolactin receptor,
preferably, the extracellular domain includes amino acids 1 to 210 of SEQ ID NO: 99 or 101,
more preferably, the antibody comprises: three HCDRs of the heavy chain variable region shown by any one of SEQ ID NOs: 1 to 23, and/or three LCDRs of the light chain variable region shown by any one of SEQ ID NOs: 24 to 41.

2. The antibody or antigen-binding fragment thereof according to claim 1, wherein
the HCDR1 of the antibody comprises any one selected from SEQ ID NOs: 42 to 52, or a sequence having at least 85% sequence identity thereto, and/or
the HCDR2 of the antibody comprises any one selected from SEQ ID NOs: 53 to 66, or a sequence having at least 85% sequence identity thereto, and/or
the HCDR3 of the antibody comprises any one selected from SEQ ID NOs: 67 to 75, or a sequence having at least 85% sequence identity thereto, and/or
the LCDR1 of the antibody comprises any one selected from SEQ ID NOs: 76 to 83, or a sequence having at least 85% sequence identity thereto, and/or
the LCDR2 of the antibody comprises any one selected from SEQ ID NOs: 84 to 90, or a sequence having at least 85% sequence identity thereto, and/or
the LCDR3 of the antibody comprises any one selected from SEQ ID NOs: 91 to 98, or a sequence having at least 85% sequence identity thereto,
preferably, the antibody comprises HCDR1, HCDR2, and HCDR3 of an antibody shown in any one row of Table 1, or a sequence having at least 85% sequence identity thereto, and/or the antibody comprises LCDR1, LCDR2 and LCDR3 of an antibody shown in any one row of Table 1, or a sequence having at least 85% sequence identity thereto,
more preferably, the antibody comprises HCDR1, HCDR2, HCDR3, LCDR1, LCDR2 and LCDR3 of the antibody shown in any one row of Table 1.

3. The antibody or antigen-binding fragment thereof according to claim 1 or 2, wherein the light chain variable region of the antibody comprises a murine or human light chain FR region, and the heavy chain variable region of the antibody comprises a murine or human heavy chain FR region,
preferably, FR1, FR2, FR3 and FR4 of the heavy chain variable region of the antibody are each independently selected from FR1, FR2, FR3 and FR4 of the heavy chain variable region shown by any one of SEQ ID NOs: 1 to 23, and/or, FR1, FR2, FR3 and FR4 of the light chain variable region of the antibody are each independently selected from FR1, FR2, FR3 and FR4 of the light chain variable region shown by any one of SEQ ID NOs: 24 to 41,
more preferably, the antibody comprises VH, VL, or VH and VL of an antibody shown in any one row of Table 1, or a sequence having at least 85% sequence identity thereto.

4. The antibody or antigen-binding fragment thereof according to claim 1 or 2, wherein
the antibody further comprises a heavy chain constant region and/or a light chain constant region, and/or
the antibody is a multispecific antibody, and/or
the antibody is a monoclonal antibody, and/or
the antibody is a chimeric antibody or a fully human antibody.

5. A fusion protein or antibody conjugate comprising the antibody or antigen-binding fragment thereof according to any one of claims 1 to 4.

6. A polynucleotide, selected from:
(1) the coding sequence of the antibody or antigen-binding fragment thereof according to any one of claims 1 to 4, or the fusion protein or antibody conjugate according to claim 5;
(2) a complement sequence of (1).

7. A nucleic acid construct, which expresses the antibody or antigen-binding fragment thereof according to any one of claims 1 to 4, or expresses the fusion protein or antibody conjugate according to claim 5, or comprises the polynucleotide according to claim 6,
preferably, the nucleic acid construct is a vector.

8. A phage comprising the antibody or antigen-binding fragment thereof according to any one of claims 1 to 4, or a library comprising the phage.

9. A host cell, which:
(1) expressing and/or secreting the antibody or antigen-binding fragment thereof according to any one of claims 1 to 4;
(2) comprises the polynucleotide according to claim 6; and/or
(3) the host cell comprises the nucleic acid construct according to claim 7,
preferably, the host cell is a mammalian cell.

10. A method for producing an antibody or an antigen-binding fragment thereof, comprising: culturing the host cell according to claim 9 under conditions suitable for producing the antibody or the antigen-binding fragment thereof, and optionally purifying the antibody or the antigen-binding fragment thereof from the culture.

11. A pharmaceutical composition, comprising the antibody or antigen-binding fragment thereof according to any one of claims 1 to 4, fusion protein, antibody conjugate, polynucleotide, nucleic acid construct, phage or host cell, and a pharmaceutically acceptable excipient.

12. Use of the antibody or antigen-binding fragment thereof according to any one of claims 1 to 4, the fusion protein or antibody conjugate according to claim 5, the polynucleotide according to item 6, the nucleic acid construct according to claim 7 or the host cell according to claim 9 in the preparation of a medicament for preventing or treating a disease,
preferably, the diseases include: cancer, endometriosis, alopecia, osteoporosis, obesity, benign lesions caused by PRLR activation,
more preferably, the cancer is a PRLR-related cancer,
further preferably, the cancer is selected from the group consisting of: ovarian cancer, melanoma, prostate cancer, intestinal cancer, gastric cancer, esophageal cancer, breast cancer, lung cancer, kidney cancer, pancreatic cancer, uterine cancer, liver cancer, bladder cancer, cervical cancer, oral cancer, brain cancer, testicular cancer, skin cancer, thyroid cancer and hematological malignancies.

13. A kit for detecting PRLR, for evaluating drug treatment effects or diagnosing cancer, wherein the kit comprises the antibody or antigen-binding fragment thereof according to any one of claims 1 to 4, the fusion protein or antibody conjugate according to claim 5, the polynucleotide according to claim 6, the nucleic acid construct according to claim 7, the bacteriophage according to claim 8, or the host cell according to claim 9,
preferably, the kit further comprises a reagent for detecting the binding of PRLR to an antibody or antigen-binding fragment thereof, fusion protein, or antibody conjugate.

14. Use of the antibody or antigen-binding fragment thereof according to any one of claims 1 to 4, the fusion protein or antibody conjugate according to claim 5, the polynucleotide according to claim 6, the nucleic acid construct according to claim 7, the bacteriophage described in claim 8 or the host cell according to claim 9 in the preparation of a kit for detecting PRLR in a sample, evaluating the efficacy of drug treatment or diagnosing cancer.
